# EUROPEAN PATENT APPLICATION

(11) **EP 2 186 518 A1**
(43) Date of publication of application: **19.05.2010**
(21) Application number: 10000985.1
(22) Date of filing: 17.11.2005
(51) Int. Cl.: A61K 31/7004, A61P 25/28, A61K 31/047

(54) **Composition and methods for treatment of disorders of protein aggregation**

(30) Priority: 17.11.2004 US 628840 P
(62) Divisional of application: 05808109.2
(71) Applicant: McLaurin, Joanne, East York, Ontario M4J 2C4 (CA)
(72) Inventor: McLaurin, Joanne, East York, Ontario M4J 2C4 (CA)
(74) Representative: Holliday, Louise Caroline

(57) **Abstract**

The invention provides compositions, methods and uses comprising a scyllo-inositol compound that provide beneficial effects in the treatment of a disorder and/or disease including a disorder in protein folding and/or aggregation, and/or amyloid formation, deposition, accumulation, or persistence.

## Description

### FIELD OF THE INVENTION

The invention relates generally to scyllo-inositol compounds and compositions, and methods and uses of the compositions, in particular methods for treating diseases characterized by abnormal protein folding or aggregation or amyloid formation, desposition, accumulation or persistence.

### BACKGROUND OF THE INVENTION

Multiple lines of evidence suggest that the accumulation of neurotoxic oligomeric/protofibrillar aggregates of amyloid β-peptide (Aβ) is a central event in the pathogenesis of Alzheimer disease (AD) [1,2]. This has led to attempts to develop therapies based upon blocking the generation of Aβ (with β- or γ-secretase inhibitors), accelerating its removal, or preventing its aggregation and toxicity. The potential utility of anti-Aβ therapies for AD has received tentative support from a clinical trial of a vaccine, which suggested clinical and neuropathological improvement in a small cohort of AD patients [3,4]. However, the anti-Aβ vaccine also induced a T-cell-mediated meningo-encephalitis in some patients which renders this particular vaccine unsuitable for widespread clinical use [5]. Nevertheless, Aβ vaccines have been shown in some mouse models to act via antibody-mediated inhibition of Aβ fibrillogenesis and toxicity [6-8]. Thus, it would be desirable to identify small molecule inhibitors of Aβ-aggregation that would avoid the potential risks of immunotherapy.

### SUMMARY OF THE INVENTION

The invention provides a composition, in particular a pharmaceutical composition, comprising a scyllo-inositol compound that provides beneficial effects in the treatment of a disorder and/or disease described herein, in particular a disorder in protein folding and/or aggregation, and/or amyloid formation, deposition, accumulation, or persistence. In an aspect the invention provides a pharmaceutical composition, comprising one or more scyllo-inositol compound that provides beneficial effects, in particular sustained beneficial effects, following treatment. The beneficial effects provided by a composition of the invention can include enhanced therapeutic effects, in particular sustained therapeutic effects.

The invention also provides a pharmaceutical composition intended for administration to a subject to provide beneficial effects, in particular sustained beneficial effects, comprising a scyllo-inositol compound, in particular a pure scyllo-inositol compound, more particularly a substantially pure scyllo-inositol compound, optionally together with one or more pharmaceutically acceptable carriers, excipients, or vehicles.

The invention also provides a pharmaceutical composition for the treatment of a disorder and/or disease comprising a therapeutically effective amount of a scyllo-inositol compound to provide a sustained beneficial effect in a pharmaceutically acceptable carrier, excipient, or vehicle.

In an aspect, a pharmaceutical composition comprising a scyllo-inositol compound is provided which has been adapted for administration to a subject to provide sustained beneficial effects to treat a disorder and/or disease. In an embodiment, the composition is in a form such that administration to a subject suffering from a disorder and/or disease results in inhibition, reduction, or reversal of Aβ fibril assembly or aggregation, Aβ toxicity, abnormal protein folding, aggregation, amyloid formation, deposition, accumulation or persistence, and/or amyloid lipid interactions, and/or acceleration of disassembly of preformed fibrils. In particular, the composition is in a form that results in disruption of aggregating Aβ or Aβ oligomers, increased or restored long term potentiation, maintenance of synaptic function; and/or reduced cerebral accumulation of amyloid β, deposition of cerebral amyloid plaques, soluble Aβ oligomers in the brain, glial activity, inflammation, and/or cognitive decline in the subject, in particular for a sustained period of time after cessation of treatment.

The present invention is directed to compositions comprising a scyllo-inositol compound that provides beneficial effects, in particular sustained beneficial effects, in the treatment of a disorder and/or disease in particular, a disorder and/or disease characterized by amyloid deposition, more particularly Alzheimer's disease.

In another aspect, the invention features a composition comprising a scyllo-inositol compound in a dosage effective for disrupting aggregation of Aβ or Aβ oligomers, increasing or restoring long term potentiation and/or maintenance of synaptic function, and/or for reducing cerebral accumulation of amyloid β, deposition of cerebral amyloid plaques, soluble Aβ oligomers in the brain, glial activity, inflammation, and/or cognitive decline in the subject, in particular for a sustained period following administration of the compound. The composition can be in a pharmaceutically acceptable carrier, excipeint, or vehicle.

The invention additionally provides a method of preparing a stable pharmaceutical composition comprising one or more scyllo-inositol compound adapted to provide beneficial effects, preferably sustained beneficial effects, following treatment. The invention further provides a method of preparing a stable pharmaceutical composition comprising a therapeutically effective amount of one or more pure, in particular substantially pure, scyllo-inositol compound adapted to provide beneficial effects, preferably sustained beneficial effects, following treatment. After compositions have been prepared, they can be placed in an appropriate container and labelled for treatment of an indicated condition. For administration of a composition of the invention, such labelling would include amount, frequency, and method of administration.

A scyllo-inositol compound for use in the present invention may be in the form of a prodrug that is converted *in vivo* to an active compound. By way of example, a scyllo-inositol compound may comprise a cleavable group that is cleaved after administration to a subject to provide an active (e.g. therapeutically active) compound, or an intermediate compound that subsequently yields the active compound. The cleavable group may be an ester that can be removed either enzymatically or non-enzymatically.

A scyllo-inositol compound for use in the present invention may optionally comprise a carrier interacting with the compound. A carrier may include a polymer, carbohydrate, or peptide, or combinations thereof. A carrier may be substituted, for example, with one or more alkyl, halo, thiol, hydroxyl, or amino group.

In an aspect, the invention provides a dietary supplement composition comprising one or more scyllo-inositol compound or nutraceutically acceptable derivatives thereof. In an aspect, the invention provides a dietary supplement for mammalian consumption, particularly human consumption for the purpose of improving memory comprising a scyllo-inositol compound or nutraceutically acceptable derivatives thereof. In another aspect, the invention provides a supplement comprising a scyllo-inositol compound or nutraceutically acceptable derivatives thereof for slowing the deterioration of mental processes and improving memory, in particular short-term memory, of individuals who have taken the supplement. A dietary supplement of the invention is preferably pleasant tasting, effectively absorbed into the body and provides substantial therapeutic effects.

The invention also provides methods to make commercially available formulations which contain a scyllo-inositol compound.

In an aspect, scyllo-inositol compounds, in particular pure or substantially pure scyllo-inositol compounds, and compositions of the invention may be administered therapeutically or prophylactically to treat disorders and/or diseases disclosed herein, in particular a disorder and/or disease associated with amyloid formation, aggregation or deposition. While not wishing to be bound by any particular theory, the compounds and compositions may act to ameliorate the course of a disease using without limitation one or more of the following mechanisms: preventing, reducing, reversing, and/or inhibiting Aβ fibril or Aβ oligomer assembly or aggregation, Aβ toxicity, Aβ42 levels, abnormal protein folding or aggregation, amyloid formation, deposition, accumulation or persistence, and/or amyloid interactions; preventing, reducing, reversing, and/or inhibiting neurodegeneration or cellular toxicity induced by Aβ; accelerating disassembly of preformed fibrils; disrupting or dissociating aggregating Aβ or Aβ oligomers; increasing or restoring long term potentiation; maintaining synaptic function; enhancing clearance of Aβ from the brain; increasing degradation of Aβ; and/or, preventing, reducing, reversing, and/or inhibiting cerebral accumulation of amyloid β, deposition of cerebral amyloid plaques, soluble Aβ oligomers in the brain, glial activity, inflammation, and/or cognitive decline.

The invention also contemplates the use of a composition comprising at least one scyllo-inositol compound for the preparation of a medicament for preventing and/or treating disorders and/or diseases. The invention additionally provides uses of a pharmaceutical composition of the invention in the preparation of medicaments for the prevention and/or treatment of disorders and/or diseases.

The invention provides a method for treating and/or preventing disorders and/or diseases in a subject comprising administering to the subject a therapeutically effective amount of one or more scyllo-inositol compound to provide beneficial effects. In an aspect the invention provides a treatment which results in sustained beneficial effects following treatment.

This invention also includes a regimen for supplementing a healthy human's diet by administering a scyllo-inositol compound or a dietary supplement comprising a scyllo-inositol compound or a nutraceutically acceptable derivative thereof, and an acceptable carrier, to the human. The invention further includes a regimen for supplementing a healthy human's diet by administering daily to the human a scyllo-inositol compound or a nutraceutically acceptable derivative thereof.

The invention also provides a kit comprising one or more scyllo-inositol compound or a pharmaceutical composition of the invention. In an aspect, the invention provides a kit for preventing and/or treating a disorder and/or disease, containing a composition comprising one or more scyllo-inositol compound, a container, and instructions for use. The composition of the kit can further comprise a pharmaceutically acceptable carrier, excipient, or vehicle.

These and other aspects, features, and advantages of the present invention should be apparent to those skilled in the art from the following drawing and detailed description.

Further aspects of the invention are set out below in the following numbered paragraphs:-
1. A pharmaceutical composition comprising one or more scyllo-inositol compound of the formula Ia or Ib 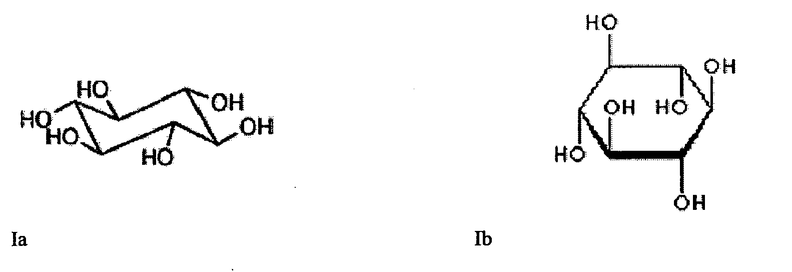 or a compound of the formula Ia or Ib wherein one, two or three hydroxyl groups are replaced by substituents with retention of configuration, or pharmaceutically acceptable salts thereof, in a therapeutically effective amount to provide beneficial effects in the treatment of a disorder characterized by abnormal protein folding and/or aggregation, and/or amyloid formation, deposition, accumulation, or persistence.
2. A pharmaceutical composition of paragraph 1 comprising a compound of the formula Ia or Ib wherein one, two or three hydroxyl groups are replaced by hydrogen, alkyl, acyl, alkenyl, cycloalkyl, halogen, -NHR¹ wherein R¹ is hydrogen, acyl, alkyl or -R²R³ wherein R² and R³ are the same or different and represent acyl or alkyl; -PO₃H₂; -SR⁴ wherein R⁴ is hydrogen, alkyl, or -O₃H; and -OR³ wherein R³ is hydrogen, alkyl, or -SO₃H.
3. A pharmaceutical composition of paragraph 1 comprising a compound of the formula Ia or Ib wherein one or more of the hydroxyl groups are replaced with alkyl, acyl, alkenyl, -NHR¹ wherein R¹ is hydrogen, acyl, alkyl or -R²R³ wherein R² and R³ are the same or different and represent acyl or alkyl; -SR⁴ wherein R⁴ is hydrogen, alkyl, or -O₃H, or -OR³ wherein R³ is hydrogen, alkyl, or -SO₃H.
4. A pharmaceutical composition according to paragraph 1 comprising a compound of the formula Ia or Ib wherein one or more of the hydroxyl groups are replaced with -SR⁴ wherein R⁴ is hydrogen, alkyl, -O₃H or - SO₃H.
5. A pharmaceutical composition according to any preceding paragraph wherein the compound comprises a carrier interacting with the compound.
6. A pharmaceutical composition according to any preceding paragraph wherein the compound is in the form of a prodrug.
7. A pharmaceutical composition of any preceding paragraph wherein the beneficial effects include one or more ofthe following: reduction, reversal or inhibition of Aβ fibril assembly or aggregation, Aβ toxicity, Aβ42 levels, abnormal protein folding, aggregation, amyloid formation, deposition, accumulation or persistence, and/or amyloid lipid interactions, and/or acceleration of disassembly of preformed fibrils.
8. A pharmaceutical composition of any preceding paragraph wherein the beneficial effects include one or more of the following: disruption of aggregated Aβ or Aβ oligomers; increased or restored long term potentiation; maintenance of synaptic function; inhibition, reduction or reversal of Aβ-induced progressive cognitive decline and cerebral amyloid plaque pathology; improved cognition; increased lifespan; reduced cerebral accumulation of Aβ; reduced deposition of cerebral amyloid plaques; reduced soluble Aβ oligomers in the brain; reduced glial activity; reduced inflammation; and/or cognitive decline.
9. A pharmaceutical composition according to any preceding paragraph wherein the disorder is a condition of the central or peripheral nervous system or a systemic organ that results in the deposition of proteins, protein fragments, and peptides in beta-pleated sheets, fibrils, and/or aggregates or oligomers.
10. A pharmaceutical composition according to paragraph 8 wherein the disorder is characterized by amyloid deposition.
11. A pharmaceutical composition according to paragraph 1 wherein the disorder is Alzheimer's disease, Down's syndrome, dementia pugilistica, multiple system atrophy, inclusion body myositosis, hereditary cerebral hemorrhage with amyloidosis of the Dutch type, Nieman-Pick disease type C, cerebral β-amyloid angiopathy, dementia associated with cortical basal degeneration, amyloidosis of type 2 diabetes, amyloidosis of chronic inflammation, amyloidosis of malignancy and Familial Mediterranean Fever, amyloidosis of multiple myeloma and B-cell dyscrasias, amyloidosis of the prion diseases, Creutzfeldt-Jakob disease, Gerstmann-Straussler syndrome, kuru, and scrapie, amyloidosis associated with carpal tunnel syndrome, senile cardiac amyloidosis, familial amyloidotic polyneuropathy, or amyloidosis associated with endocrine tumors.
12. A pharmaceutical composition according to any preceding paragraph for oral administration.
13. A pharmaceutical composition according to any preceding paragraph wherein the disease is Alzheimer's disease.
14. A pharmaceutical composition according to any preceding paragraph for oral administration of one or more.scyllo-inositol compound for treatment of Alzheimer's disease.
15. A pharmaceutical composition according to any preceding paragraph comprising a pharmaceutically acceptable carrier, excipient or vehicle.
16. A stable oral pharmaceutical composition for treatment of a disorder characterized by abnormal protein folding and/or aggregation, and/or amyloid formation, deposition, accumulation, or persistence comprising a substantially pure scyllo-inositol compound of the formula Ia or Ib as defined in paragraph 1, 2, 3, or 4.
17. A pharmaceutical composition according to any preceding paragraph comprising a scyllo-inositol compound produced using microbial process steps.
18. A method for treating a disorder characterized by abnormal protein folding and/or aggregation, and/or amyloid formation, deposition, accumulation, or persistence in a subject comprising administering to a subject a therapeutically effective amount of one or more scyllo-inositol compound as defined in any preceding paragraph to provide beneficial effects, preferably sustained beneficial effects following treatment.
19. A method according to paragraph 18 wherein the disorder is characterized by amyloid deposition.
20. A method of paragraph 18 wherein the wherein the beneficial effects include one or more of the following: disruption of aggregated Aβ or Aβ oligomers; increased or restored long term potentiation; maintenance of synaptic function; inhibition, reduction or reversal of Aβ-induced progressive cognitive decline and cerebral amyloid plaque pathology; improved cognition; increased lifespan; reduced cerebral accumulation of Aβ; reduced deposition of cerebral amyloid plaques; reduced soluble Aβ oligomers in the brain; reduced glial activity; reduced inflammation; and/or cognitive decline.
21. A method of delaying the progression of a disorder characterized by abnormal protein folding and/or aggregation, and/or amyloid formation, deposition, accumulation, or persistence comprising administering a therapeutically effective amount of a scyllo-inositol compound as defined in any preceding paragraph or a composition according to any preceding paragraph.
22. A method of reducing, reversing or inhibiting amyloid deposition and neuropathology after the onset of cognitive deficits and amyloid plaque neuropathology in a subject comprising administering to the subject a therapeutically effective amount of a scyllo-inositol compound as defined in any preceding paragraph or a composition according to any preceding paragraph.
23. A method of improving cognitive deficits in a subject suffering from Alzheimer's disease comprising administering to the subject a therapeutically effective amount of a scyllo-inositol compound as defined in any preceding paragraph or a composition according to any preceding paragraph.
24. A method for increasing or maintaining synaptic function in a subject comprising administering a therapeutically effective amount of one or more scyllo-inositol compound as defined in any preceding paragraph, a pharmaceutically acceptable salt thereof, or a composition according to any preceding paragraph.
25. A method according to any preceding paragraph wherein the composition is formulated for oral administration.
26. Use of a scyllo-inositol compound as defined in any preceding paragraph or a composition according to any preceding paragraph for preparation of a medicament for preventing and/or treating a disorder characterized by abnormal protein folding and/or aggregation, and/or amyloid formation, deposition, accumulation, or persistence.
27. Use as claimed in paragraph 26 wherein the medicament provides one or more beneficial effects following treatment.
28. Use as claimed in paragraph 27 wherein the beneficial effects include one or more of the following: disruption of aggregated Aβ or Aβ oligomers; increased or restored long term potentiation; maintenance of synaptic function; inhibition, reduction or reversal of Aβ-induced progressive cognitive decline and cerebral amyloid plaque pathology; improved cognition; increased lifespan; reduced cerebral accumulation of Aβ; reduced deposition of cerebral amyloid plaques; reduced soluble Aβ oligomers in the brain; reduced glial activity; reduced inflammation; and/or cognitive decline.
29. Use of a scyllo-inositol compound as defined in any preceding paragraph or a composition according to any preceding paragraph for preparation of a pharmaceutical composition to be employed through oral administration for treatment of a disorder characterized by abnormal protein folding and/or aggregation, and/or amyloid formation, deposition, accumulation, or persistence.
30. Use according to any preceding paragraph comprising a scyllo-inositol compound produced using microbial process steps.
31. A kit comprising one or more scyllo-inositol compound as defined in any preceding paragraph or a pharmaceutical composition according to any preceding paragraph, a container, and instructions for use.

### DESCRIPTION OF THE DRAWINGS

The invention will be better understood with reference to the drawings in which:

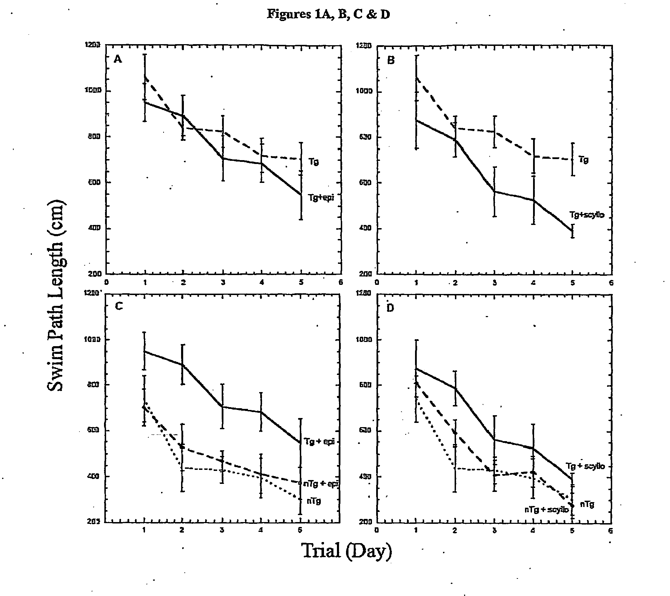

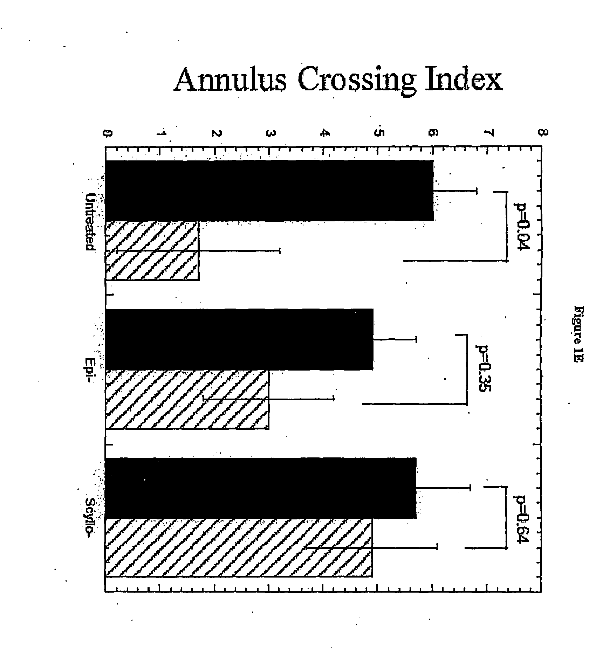

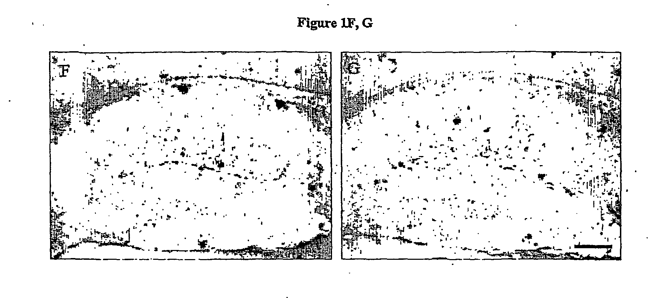

Figure 1. Spatial reference memory test in six month old mice following 28 days of treatment, beginning at five months of age (n=10 mice per treatment arm) was performed. The performance of epi-cyclohexanehexol treated TgCRND8 mice was not different from untreated TgCRND8 littermates (p=0.27; Figure 1A) and remained impaired with respect to non-Tg littermates (F_{1,14}=11.7, p=0.004; Figure 1C). In contrast, scyllo-cyclohexanehexol treated TgCRND8 mice were significantly better than untreated TgCRND8 littermates (p=0.01; Figure 1B) and were indistinguishable from non-Tg littermates (F_{1,13}=2.9, p=0.11; Figure 1D). The probe trial, using annulus crossing index, demonstrated that scyllo-cyclohexanehexol treated mice were not statistically different from non-Tg littermates (p=0.64; Figure 1E). Vertical bars represent s.e.m. After one month of scyllo-cyclohexanehexol treatment, mice had a lower plaque burden compared to control animals with a high plaque burden in the hippocampus (Figure 1F, Figure 1G). Plaque burden was identified using anti-Aβ antibody (brown) and astrocytes are labeled using anti-GFAP antibody (red). Scale bar 300 µm.

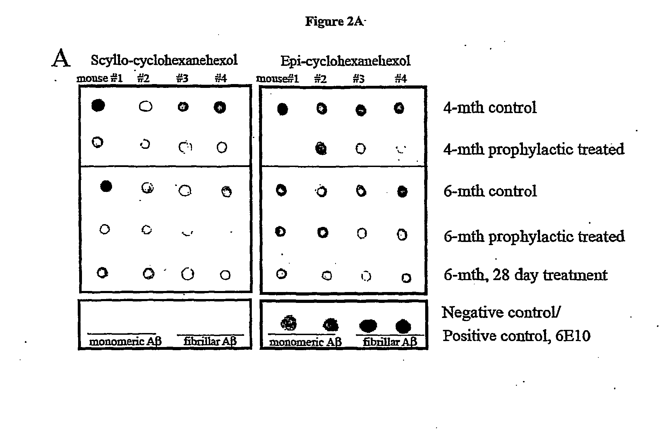

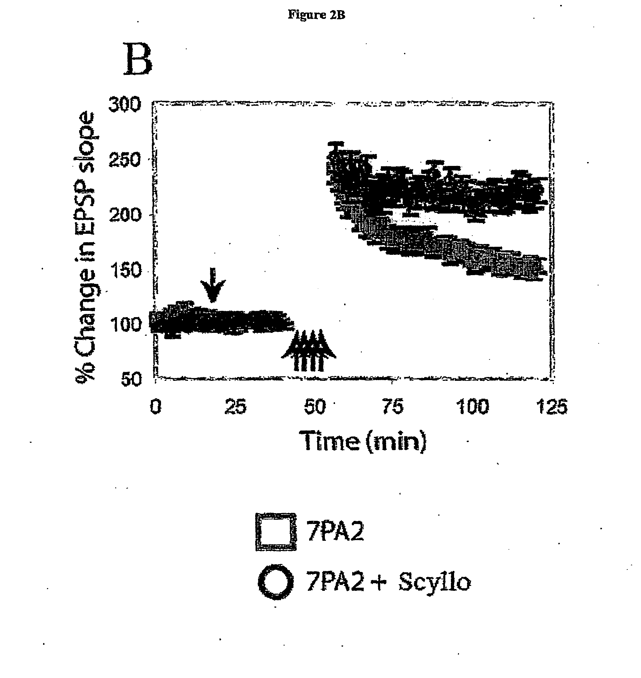

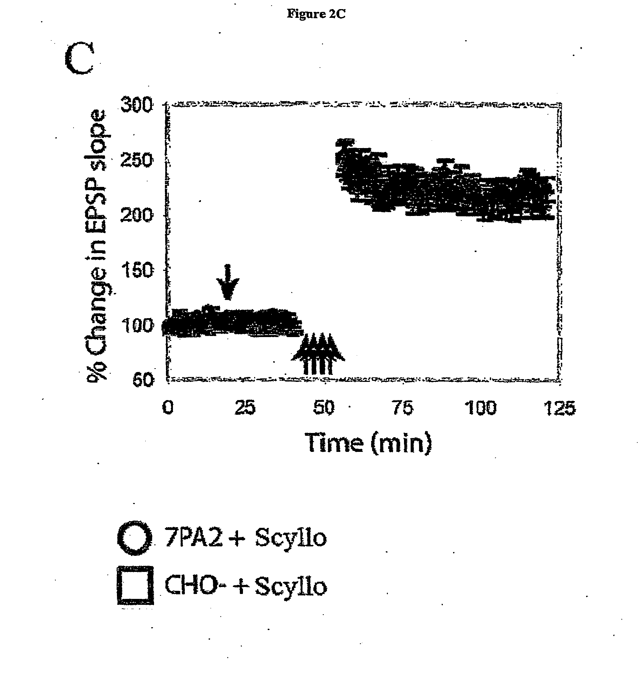

Figure 2. Dot blot analyses of soluble oligomeric Aβ in scyllo-cyclohexanehexol and epi-cyclohexanehexol treated and untreated TgCRND8 mice (Figure 2A). Soluble proteins isolated from 4 representative four and six month old untreated and treated TgCRND8 mice from the prophylactic study, and from the five month old treatment groups, untreated and treated were applied to nitrocellulose and probed with oligomer-specific antibody followed by re-probing with 6E10. Synthetic Aβ42, monomeric (bottom row: lane 1 and 2) and fibrillar (lane 3 and 4) were used as negative controls for the oligomer-specific antibody, which only recognizes soluble aggregates. 6E10 recognises all Aβ species (bottom lane, right four lanes). Long-term potentiation is blocked by soluble Aβ oligomers (Figure 2B; green squares) and rescued by scyllo-cyclohexanehexol treatment (Figure 2B; blue circles). LTP is unaffected by scyllo-cyclohexanehexol treated 7PA2 culture medium which contains Aβ oligomers (Figure 2C; red squares; same data as in Figure 2B) and plain CHO medium which lacks oligomers (Figure 2C; blue circles).

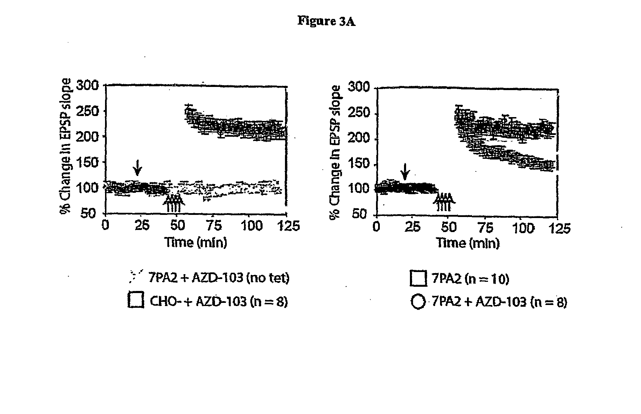

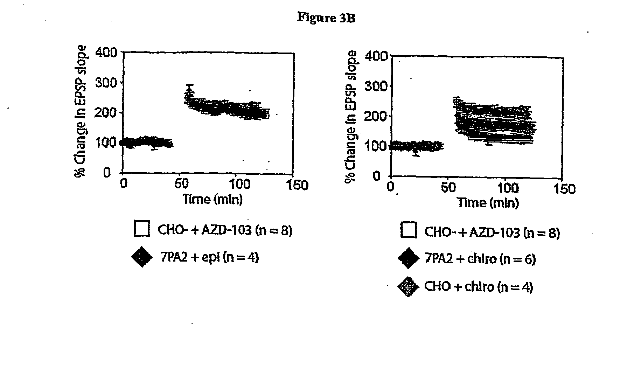

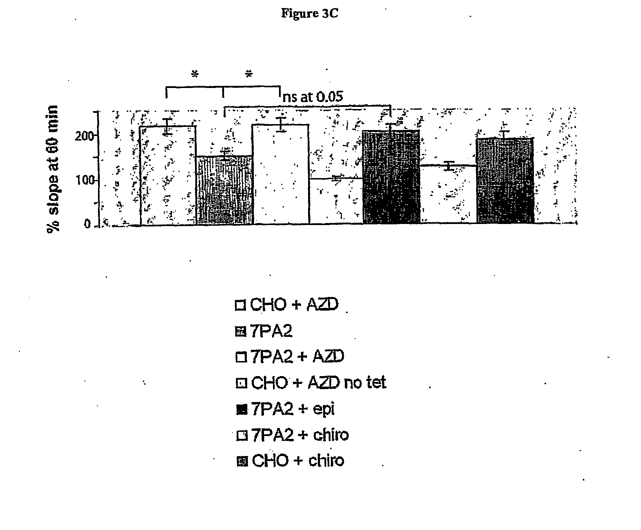

Figure 3 are graphs showing the impact of AZD 103 on Aβ-dependent inhibition of induction of long-term potentiation. Following repeated stimulation ("tetanus": multi-arrows), the extent of the field potential following a single stimulation (single arrow) is increased. This can be quantified by recording of the % change of the slope of the field potential (EPSP slope). Figure 3 shows the % change in EPSP slope with time, following perfusion of the hippocampal slices with pre-incubated mixture of 1.25µM AZD-103 + CHO CM (conditioned medium)(CHO cells do not secrete Aβ oligomers), or 1.25µM AZD-103 + 7PA2 CM (which do secrete A□ oligomers), and 7PA2 CM alone (in each case, CM and AZD103 were pre-incubated together for 30 minutes prior to perfusion) (Figure 3A); the % change in EPSP slope with time following perfusion ofhippocampal slices with pre-incabated mixtures of 7PA2 CM with epi-inositol or chiro-inositol (Figure 3B); a comparison of the % change in EPSP slope 60 minutes after tetanus, when the slices had been perfused with pre-incubated mixture of 7PA2 CM alone, or with 1.25µM AZD-103, epi-inositol, and chiro-inositol; and of CHO CM with AZD103 and chiro-inositol (Figure 3C).
Figure 4 are Western blots illustrating that the application of AZD-103 to 7PA2 CM reduced the detectability of the Aβ trimer.
Figure 5 is a dose response curve of AZD-103, evaluating its ability to prevent the inhibitory effect of Aβ on induction of LTP. Four different concentrations of AZD-103 (0.125, 0.5, 1.25, and 5.0 µM) were added to 7PA2 CM, and hippocampal slices then perfused with the mixture.
Figure 6A is a graph demonstrating the effect of time of pre-incubation of AZD103 with 7PA2 CM on the induction of LTP. The graph shows the % change in EPSP slope over time for four different pre-incubation times (15, 30, 120, and 240 minutes).
Figure 6B is a bar graph showing the % change in EPSP slope 60 minutes post tetanus for 0.5 µM AZD-103 pre-incubated with 7PA2 CM for 15, 30, 120, and 240 minutes.
Figure 6C is a graph showing % change in EPSP slope versus time following perfusion of mouse brain slices with 7PA2 CM alone, followed 20 minutes later by 0.5 µM AZD-103.
Figure 7A and 7B are Western blots showing the effect of AZD 103 on Aβ oligomers when AZD-103 is added to 7PA2 cells themselves, directly prior to conditioning (pre-cond), and when AZD-103 is added to 7PA2 CM (post-cond).
Figure 7C is a graph showing AZD-103 effects on oligomers assessed directly.
Figure 7D is a graph showing AZD-103 effects on oligomers normalized to levels of APP.
Figure 7 E is a graph showing AZD-103 effects on oligomers normalized to levels of Aβ monomers.
Figure 8 is a graph showing % change in EPSP slope versus time following perfusion of brain slices with CM from 7PA2 cells that were incubated themselves with 0.5µM AZD-103 (pre-conditioning).
Figure 9 is a graph showing alleviation of Aβ-induced acute cognitive dysfunction by preincubation of Aβ with AZD103. 100% error rate is set by the number of errors made by the animals at baseline. Error rate following infusion of Aβ alone, Aβ + AZD103 , and AZD103 alone is shown.
Figure 10 is a graph showing alleviation of Aβ-induced acute cognitive dysfunction by oral administration of AZD103. 100% error rate is set by the number of errors made by the animals at baseline. Error rates are shown for animals receiving icv infusion of Aβ, when treated orally with 0, 30, 100 and 300 mg/kg/day AZD 103.

### DETAILED DESCRIPTION OF EMBODIMENTS

### Glossary

Numerical ranges recited herein by endpoints include all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about." The term "about" means plus or minus 0.1 to 50%, 5-50%, or 10-40%, preferably 10-20%, more preferably 10% or 15%, of the number to which reference is being made. Further, it is to be understood that "a," "an," and "the" include plural referents unless the content clearly dictates otherwise. Thus, for example, reference to a composition containing "a compound" includes a mixture of two or more compounds.

The terms "administering" and "administration" refer to the process by which a therapeutically effective amount of a compound or composition contemplated herein is delivered to a subject for prevention and/or treatment purposes. Compositions are administered in accordance with good medical practices taking into account the subject's clinical condition, the site and method of administration, dosage, patient age, sex, body weight, and other factors known to physicians.

The term "treating" refers to reversing, alleviating, or inhibiting the progress of a disorder and/or disease, or one or more symptoms of such disorder and/or disease, to which such term applies. Depending on the condition of the subject, the term also refers to preventing a disease, and includes preventing the onset of a disease, or preventing the symptoms associated with a disease. A treatment may be either performed in an acute or chronic way. The term also refers to reducing the severity of a disease or symptoms associated with such disease prior to affliction with the disease. Such prevention or reduction of the severity of a disease prior to affliction refers to administration of a compound or composition of the present invention to a subject that is not at the time of administration afflicted with the disease. "Preventing" also refers to preventing the recurrence of a disease or of one or more symptoms associated with such disease. The terms "treatment" and "therapeutically," refer to the act of treating, as "treating" is defined above.

The terms "subject", "individual", or "patient" are used interchangeably herein and refer to an animal including a warm-blooded animal such as a mammal, which is afflicted with or suspected ofhaving or being predisposed to a disorder and/or disease disclosed herein. Mammal includes without limitation any members of the Mammalia. In aspects of the invention, the terms refer to a human. The terms also include domestic animals bred for food or as pets, including horses, cows, sheep, poultry, fish, pigs, cats, dogs, and zoo animals, goats, apes (e.g. gorilla or chimpanzee), and rodents such as rats and mice. Typical subjects for treatment include persons susceptible to, suffering from or that have suffered a disorder and/or disease disclosed herein. A subject may or may not have a genetic predisposition for a disorder and/or disease disclosed herein such as Alzheimer's disease. In some aspects, a subject shows signs of cognitive deficits and amyloid plaque neuropathology. In embodiments of the invention the subjects are suspectible to, or suffer from Alzheimer's disease.

As utilized herein, the term "healthy subject" means a subject, in particular a mammal, having no disorder and/or disease, in particular no diagnosed disease, disorder, infirmity, or ailment known to impair or otherwise diminish memory.

The term "pharmaceutically acceptable carrier(s), excipient(s), or vehicle(s)" refers to a medium which does not interfere with the effectiveness or activity of an active ingredient and which is not toxic to the hosts to which it is administered. A carrier, excipient, or vehicle includes diluents, binders, adhesives, lubricants, disintegrates, bulking agents, wetting or emulsifying agents, pH buffering agents, and miscellaneous materials such as absorbants that may be needed in order to prepare a particular composition. Examples of carriers etc include but are not limited to saline, buffered saline, dextrose, water, glycerol, ethanol, and combinations thereof. The use of such media and agents for an active substance is well known in the art.

As used herein "nutraceutically acceptable derivative" refers to a derivative or substitute for the stated chemical species that operates in a similar manner to produce the intended effect, and is structurally similar and physiologically compatible. Examples of substitutes include without limitation salts, esters, hydrates, or complexes of the stated chemical. The substitute could also be a precursor or prodrug to the stated chemical, which subsequently undergoes a reaction *in vivo* to yield the stated chemical or a substitute thereof.

The term "pure" in general means better than 90%, 92%, 95%, 97%, 98% or 99% pure, and "substantially pure" means a compound synthesized such that the compound, as made as available for consideration into a composition or therapeutic dosage of the invention, has only those impurities that can not readily nor reasonably be removed by conventional purification processes.

"Pharmaceutically acceptable salt(s)," means a salt that is pharmaceutically acceptable and has the desired pharmacological properties. By pharmaceutically acceptable salts is meant those salts which are suitable for use in contact with the tissues of a subject or patient without undue toxicity, irritation, allergic response and the like, and are commensurate with a reasonable benefit/risk ratio. Pharmaceutically acceptable salts are described for example, in S. M. Berge, et al., J. Pharmaceutical Sciences, 1977, 66:1. Suitable salts include salts that may be formed where acidic protons in the compounds are capable of reacting with inorganic or organic bases. Suitable inorganic salts include those formed with alkali metals, e.g. sodium and potassium, magnesium, calcium, and aluminum. Suitable organic salts include those formed with organic bases such as the amine bases, e.g. ethanolamine, diethanolamine, triethanolamine, tromethamine, N-methylglucamine, and the like. Suitable salts also include acid addition salts formed with inorganic acids (e.g. hydrochloride and hydrobromic acids) and organic acids (e.g. acetic acid, citric acid, maleic acid, and the alkane- and arene-sulfonic acids such as methanesulfonic acid and benezenesulfonic acid). When there are two acidic groups present, a pharmaceutically acceptable salt may be a mono-acid-mono-salt or a di-salt; and similarly where there are more than two acidic groups present, some or all of such groups can be salified.

A "combination treatment" means that the active ingredients are administered concurrently to a patient being treated. When administered in combination each component may be administered at the same time, or sequentially in any order at different points in time. Therefore, each component may be administered separately, but sufficiently close in time to provide the desired effect, in particular a beneficial, additive, or synergistic effect. The first compound may be administered in a regimen that additionally comprises treatment with the second compound. In aspects the terms refer to the administration of a scyllo-inositol compound and a second therapeutic agent optionally within one year, including separate administration of medicaments each containing one of the compounds as well as simultaneous administration whether or not the compounds are combined in one formulation or whether they are in separate formulations.

"Detectable substance" includes without limitation radioisotopes (e.g., ³H, ¹⁴C, ³⁵S, ¹²⁵I, ¹³¹I), fluorescent labels (e.g., FITC, rhodamine, lanthanide phosphors), luminescent labels such as luminol; enzymatic labels (e.g., horseradish peroxidase, beta-galactosidase, luciferase, alkaline phosphatase, acetylcholinesterase), biotinyl groups (which can be detected by marked avidin e.g., streptavidin containing a fluorescent marker or enzymatic activity that can be detected by optical or colorimetric methods), predetermined polypeptide epitopes recognized by a secondary reporter (e.g., leucine zipper pair sequences, binding sites for secondary antibodies, metal binding domains, or epitope tags). In some embodiments, labels are attached via spacer arms of various lengths to reduce potential steric hindrance.

A "beneficial effect" refers to an effect of a compound of the invention or composition thereof in certain aspects of the invention, including favorable pharmacological and/or therapeutic effects, and/or improved biological activity. In aspects of the invention, the beneficial effects include without limitation prevention, reduction, reversal or inhibition of Aβ fibril assembly or aggregation, Aβ toxicity, Aβ42 levels, abnormal protein folding, aggregation, amyloid formation, deposition, accumulation or persistence, and/or amyloid lipid interactions, and/or acceleration of disassembly of preformed fibrils. In particular embodiments of the invention, the beneficial effects include but are not limited to one or more of the following: disruption of aggregated Aβ or Aβ oligomers; increased or restored long term potentiation; maintenance of synaptic function; inhibition, reduction or reversal of Aβ-induced progressive cognitive decline and cerebral amyloid plaque pathology; improved cognition; increased lifespan; reduced cerebral accumulation of Aβ; reduced deposition of cerebral amyloid plaques; reduced soluble Aβ oligomers (e.g. Aβ42) in the brain; reduced glial activity; reduced inflammation; and/or cognitive decline. In an aspect, a beneficial effect is a favourable characteristic of a composition/formulation of the invention includes enhanced stability, a longer half life, and/or enhanced uptake and transport across the blood brain barrier. In some aspects, a beneficial effect of a composition of the invention is rapid brain penetrance, in particular brain penetrance within 1-6, 1-5, 1-4, 1-3 or 1-2 hours of administration.

The beneficial effect may be a statistically significant effect in terms of statistical analysis of an effect of a scyllo-inositol compound versus the effects without the compound. "Statistically significant" or "significantly different" effects or levels may represent levels that are higher or lower than a standard. In embodiments of the invention, the difference may be 1.5, 2, 3, 4, 5, 6, 7, 8, 9, 10, 15, 20, 25 or 50 times higher or lower compared with the effect obtained without a scyllo-inositol compound.

"Therapeutically effective amount" relates to the amount or dose of an active compound or composition of the invention that will provide or lead to one or more desired beneficial effects, in particular, one or more sustained beneficial effects. A therapeutically effective amount of a substance can vary according to factors such as the disease state, age, sex, and weight of the individual, and the ability of the substance to elicit a desired response in the individual. A dosage regimen may be adjusted to provide the optimum therapeutic response (e.g. one or more beneficial effect, in particular a sustained beneficial effect). For example, several divided doses may be administered daily or the dose may be proportionally reduced as indicated by the exigencies of the therapeutic situation.

"A scyllo-inositol compound" is understood to refer to any compound, which fully or partially, directly or indirectly, provides one or more beneficial effects described herein. A scyllo-inositol compound that can be used in the invention has the base structure of the formula Ia or Ib:

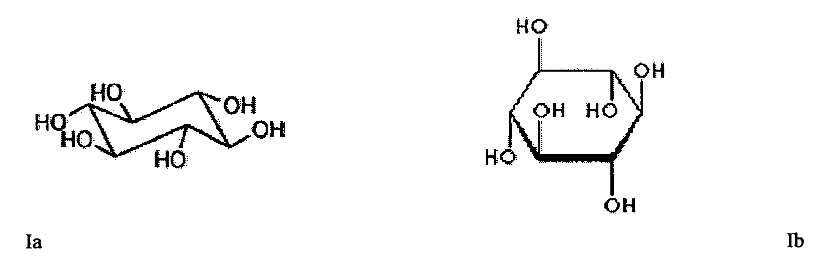

A scyllo-inositol compound includes a functional derivative of a compound of the formula Ia or Ib. A "functional derivative" refers to a compound that possesses a biological activity (either functional or structural) that is substantially similar to the biological activity of scyllo-inositol of the formula Ia or Ib. The term "functional derivative" is intended to include "variants" "analogs" or "chemical derivatives" of scyllo-inositol. The term "variant" is meant to refer to a molecule substantially similar in structure and function to scyllo-inositol or a part thereof. A molecule is "substantially similar" to scyllo-inositol if both molecules have substantially similar structures or if both molecules possess similar biological activity. The term "analog" refers to a molecule substantially similar in function to a scyllo-inositol molecule. The term "chemical derivative" describes a molecule that contains additional chemical moieties which are not normally a part of the base molecule.

A scyllo-inositol compound of the invention includes crystalline forms of the compound which may exist as polymorphs. Solvates of the compounds formed with water or common organic solvents are also intended to be encompassed within this invention. In addition, hydrate forms of scyllo-inositol compounds and their salts, are included within this invention.

A scyllo-inositol compound includes a compound of the formula Ia or Ib wherein one, two or three hydroxyl groups are replaced by substituents, in particular univalent substituents, with retention of configuration. Suitable substituents include without limitation hydrogen, alkyl, acyl, alkenyl, cycloalkyl, halogen, -NHR¹ wherein R¹ is hydrogen, acyl, alkyl or -R²R³ wherein R² and R³ are the same or different and represent acyl or alkyl; -PO₃H₂; -SR⁴ wherein R⁴ is hydrogen, alkyl, or -O₃H; and -OR³ wherein R³ is hydrogen, alkyl, or -SO₃H. In aspects of the invention, a scyllo-inositol compound does not include scyllo-inositol substituted with one or more phosphate group.

Particular aspects of the invention utilize scyllo-inositol compounds of the formula Ia or Ib wherein one or more of the hydroxyl groups is replaced with alkyl, acyl, alkenyl, -NHR¹ wherein R¹ is hydrogen, acyl, alkyl or -R²R³ wherein R² and R³ are the same or different and represent acyl or alkyl; -SR⁴ wherein R⁴ is hydrogen, alkyl, or -O₃H; and -OR³ wherein R³ is hydrogen, alkyl, or -SO₃H, more particularly -SR⁴ wherein R⁴ is hydrogen, alkyl, or -O₃H or -SO₃H.

In embodiments, scyllo-cyclohexanehexol (i.e., scyllo-inositol), in particular pure or substantially pure scyllo-cyclohexanehexol, is used in the compositions, methods and uses disclosed herein.

"Alkyl" refers to monovalent alkyl groups preferably having from 1 to 20 or 1 to 10 carbon atoms, and more preferably 1 to 6 carbon atoms. This term is exemplified by groups such as methyl, ethyl, n-propyl, isopropyl, n-butyl, iso-butyl, n-hexyl, and the like. An alkyl group can be a substituted alkyl.

"Substituted alkyl" refers to an alkyl group, preferably of from 1 to 10 carbon atoms, having from 1 to 5 substituents, and preferably 1 to 3 substituents, for example, alkyl, alkoxy, cycloalkyl, acyl, amino, cyano, halogen, hydroxyl, carboxyl, carboxylalkyl, keto, thioketo, thiol, thioalkoxy, aryl, hydroxyamino, alkoxyamino, and nitro.

"Alkenyl" refers to alkenyl groups preferably having from 2 to 10 carbon atoms and more preferably 2 to 6 carbon atoms and having at least 1 and preferably from 1-2 sites of alkenyl unsaturation. Preferred alkenyl groups include ethenyl (-CH=CH₂), n-propenyl (-CH₂CH=CH₂), iso-propenyl (-C(CH₃)=CH₂), and the like.

"Substituted alkenyl" refers to an alkenyl group as defined above having from 1 to 3 substituents, for example, alkyl, alkoxy, cycloalkyl, cycloalkoxy, acyl, acylamino, acyloxy, amino, aminoacyl, aminoacyloxy, cyano, halogen, hydroxyl, carboxyl, carboxylalkyl, keto, thioketo, thiol, thioalkoxy, aryl, and nitro.

"Acyl" refers to the groups alkyl-C(O)-, substituted alkyl-C(O)-, cycloalkyl-C(O)-, substituted cycloalkyl-C(O)-, aryl-C(O)-, heteroaryl-C(O)- and heterocyclic-C(O)- where alkyl, substituted alkyl, cycloalkyl, substituted cycloalkyl, aryl, substituted aryl, heteroaryl and heterocyclic are as defined herein.

"Aryl" refers to an unsaturated aromatic carbocyclic group of from 6 to 14 carbon atoms having a single ring (e.g., phenyl) or multiple condensed (fused) rings (e.g., naphthyl or anthryl). Preferred aryls include phenyl, naphthyl and the like. An aryl group may be a substituted aryl group which may include an aryl group as defined herein having from 1 to 8, 1 to 6, 1 to 4, or 1 to 3 substituents, for example, alkyl, alkoxy, cycloalkyl, acyl, amino, cyano, halogen, hydroxyl, carboxyl, carboxylalkyl, keto, thioketo, thiol, thioalkoxy, aryl, hydroxyamino, alkoxyamino, and nitro.

"Cycloalkyl" refers to cyclic alkyl groups of from 3 to 12 carbon atoms having a single cyclic ring or multiple condensed rings. Examples of cycloalkyl groups include single ring structures such as cyclopropyl, cyclobutyl, cyclopentyl, cyclooctyl, and the like, or multiple ring structures such as adamantanyl, and the like. A cycloalkyl can be a substituted cycloalkyl.

"Substituted cycloalkyl" refers to cycloalkyl groups having from 1 to 5 (in particular 1 to 3) substituents selected from the group consisting of alkoxy, cycloalkyl, substituted cycloalkyl, cycloalkenyl, acyl, acylamino, acyloxy, amino, aminoacyl, aminoacyloxy, oxyacylamino, cyano, halogen, hydroxyl, carboxyl, carboxylalkyl, keto, thioketo, thiol, thioalkoxy, aryl, aryloxy, heteroaryl, heteroaryloxy, heterocyclic, heterocyclooxy, hydroxyamino, alkoxyamino, and nitro.

"Halogen" refers to fluoro, chloro, bromo and iodo and preferably is either fluoro or chloro.

"Heteroaryl" refers to an aromatic group of from 1 to 15 carbon atoms and 1 to 4 heteroatoms selected from oxygen, nitrogen and sulfur within at least one ring (if there is more than one ring). Such heteroaryl groups can be optionally substituted with 1 to 5 substituents, for example, acyloxy, hydroxy, acyl, alkyl, alkoxy, alkenyl, alkynyl, substituted alkyl, substituted alkenyl, substituted alkynyl, amino, substituted amino, aminoacyl, acylamino, alkaryl, aryl, aryloxy, azido, carboxyl, carboxylalkyl, cyano, halo, nitro, heteroaryl, heterocyclic, aminoacyloxy, oxyacylamino, thioalkoxy, substituted thioalkoxy, thioaryloxy, and thioheteroaryloxy. Such heteroaryl groups can have a single ring (e.g., pyridyl or furyl) or multiple condensed rings (e.g., indolizinyl or benzothienyl).

"Heterocycle" or "heterocyclic" refers to a monovalent saturated or unsaturated group having a single ring or multiple condensed rings, from 1 to 15 carbon atoms and from 1 to 4 hetero atoms selected from nitrogen, sulfur or oxygen within the ring.

Heterocyclic groups can have a single ring or multiple condensed rings. Heterocyclic groups can be optionally substituted with 1 to 5 substituents, for example, alkoxy, cycloalkyl, substituted cycloalkyl, cycloalkenyl, acyl, acylamino, acyloxy, amino, aminoacyl, aminoacyloxy, oxyacylamino, cyano, halogen, hydroxyl, carboxyl, carboxylalkyl, keto, thioketo, thiol, thioalkoxy, aryl, aryloxy, heteroaryl, heteroaryloxy, heterocyclic, heterocyclooxy, hydroxyamino, alkoxyamino, or nitro.

Examples of heterocycles and heteroaryls include, without limitation, pyrrole, furan, imidazole, pyrazole, pyridine, pyrazine, pyrimidine, pyridazine, indolizine, isoindole, indole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, morpholino, piperidinyl, tetrahydrofuranyl, and the like as well as N-alkoxy-nitrogen containing heterocycles.

Scyllo-inositol compounds can be prepared using conventional processes or they may be obtained from commercial sources. A scyllo-inositol compound can be prepared using chemical and/or microbial processes. In aspects of the invention, a scyllo-inositol compound is produced by preparing a scyllo-inositol using process steps described by and M. Sarmah and Shashidhar, M., Carbohydrate Research, 2003, 338, 999-1001 or Husson, C., et al, Carbohyrate Research 307 (1998) 163-165. ; or described in WO05035774 (Hokko Chemical Industries). In particular aspects of the compositions and methods of the invention, a scyllo-inositol compound is prepared using the chemical process steps described in Husson, C., et al, Carbohyrate Research 307 (1998) 163-165. In other aspects of the compositions and methods of the invention, the scyllo-inositol compound is prepared using microbial process steps described in WO05035774 (Hokko Chemical Industries). Derivatives may be produced by introducing substituents into a scyllo-inositol using methods well known to a person of ordinary skill in the art

A scyllo-inositol compound may additionally comprise a carrier, including without limitation one or more of a polymer, carbohydrate, peptide or derivative thereof. A carrier may be substituted with substituents described herein including without limitation one or more alkyl, amino, nitro, halogen, thiol, thioalkyl, sulfate, sulfonyl, sulfenyl, sulfinyl, sulfoxide, hydroxyl groups. A carrier can be directly or indirectly covalently attached to a compound of the invention. In aspects of the invention the carrier is an amino acid including alanine, glycine, proline, methionine, serine, threonine, or asparagine. In other aspects the carrier is a peptide including alanyl-alanyl, prolyl-methionyl, or glycyl-glycyl.

A carrier also includes a molecule that targets a compound of the invention to a particular tissue or organ. In particular, a carrier may facilitate or enhance transport of a compound of the invention to the brain by either active or passive transport.

A "polymer" as used herein refers to molecules comprising two or more monomer subunits that may be identical repeating subunits or different repeating subunits. A monomer generally comprises a simple structure, low-molecular weight molecule containing carbon. Polymers can be optionally substituted. Examples of polymers which can be used in the present invention are vinyl, acryl, styrene, carbohydrate derived polymers, polyethylene glycol (PEG), polyoxyethylene, polymethylene glycol, poly-trimethylene glycols, polyvinylpyrrolidone, polyoxyethylene-polyoxypropylene block polymers, and copolymers, salts, and derivatives thereof. In particular aspects of the invention, the polymer is poly(2-acrylamido-2-methyl-1-propanesulfonic acid); poly(2-acrylamido-2-methyl,-1-propanesulfonic acid-coacrylonitrile, poly(2-acrylamido-2-methyl-1-propanesulfonic acid-co-styrene), poly(vinylsulfonic acid); poly(sodium 4-styrenesulfonic acid); and sulfates and sulfonates derived therefrom; poly(acrylic acid), poly(methylacrylate), poly(methyl methacrylate), and poly(vinyl alcohol).

A "carbohydrate" as used herein refers to a polyhydroxyaldehyde, or polyhydroxyketone and derivatives thereof. The simplest carbohydrates are monosaccharides, which are small straight-chain aldehydes and ketones with many hydroxyl groups added, usually one on each carbon except the functional group. Examples of monosaccharides include erythrose, arabinose, allose, altrose, glucose, mannose, threose, xylose, gulose, idose, galactose, talose, aldohexose, fructose, ketohexose, ribose, and aldopentose. Other carbohydrates are composed of monosaccharide units, including disaccharides, oligosaccharides, or polysaccharides, depending on the number of monosaccharide units. Disaccharides are composed of two monosaccharide units joined by a covalent glycosidic bond. Examples of disaccharides are sucrose, lactose, and maltose. Oligosaccharides and polysaccharides, are composed of longer chains of monosaccharide units bound together by glycosidic bonds. Oligosaccharides generally contain between 3 and 9 monosaccharide units and polysaccharides contain greater than 10 monosaccharide units. A carbohydrate group may be substituted at one two, three or four positions, other than the position of linkage to a compound of the formula Ia or Ib. For example, a carbohydrate may be substituted with one or more alkyl, amino, nitro, halo, thiol, carboxyl, or hydroxyl groups, which are optionally substituted. Illustrative substituted carbohydrates are glucosamine or galactosamine.

In aspects of the invention, the carbohydrate is a sugar, in particular a hexose or pentose and may be an aldose or a ketose. A sugar may be a member of the D or L series and can include amino sugars, deoxy sugars, and their uronic acid derivatives. In embodiments of the invention where the carbohydrate is a hexose, the hexose is selected from the group consisting of glucose, galactose, or mannose, or substituted hexose sugar residues such as an amino sugar residue such as hexosamine, galactosamine, glucosamine, in particular D-glucosamine (2-amino-2-doexy-D-glucose) or D-galactosamine (2-amino-2-deoxy-D-galactose). Suitable pentose sugars include arabinose, fucose, and ribose.

The term "carbohydrate" also includes glycoproteins such as lectins (e.g. concanavalin A, wheat germ agglutinin, peanutagglutinin, seromucoid, and orosomucoid) and glycolipids such as cerebroside and ganglioside.

A "peptide" for use as a carrier in the practice of the present invention includes one, two, three, four, or five or more amino acids covalently linked through a peptide bond. A peptide can comprise one or more naturally occurring amino acids, and analogs, derivatives, and congeners thereof. A peptide can be modified to increase its stability, bioavailability, solubility, etc. "Peptide analogue" and "peptide derivative" as used herein include molecules which mimic the chemical structure of a peptide and retain the functional properties of the peptide. In aspects of the invention the carrier is an amino acid such as alanine, glycine, proline, methionine, serine, threonine, histidine, or asparagine. In other aspects the carrier is a peptide such as alanyl-alanyl, prolyl-methionyl, or glycyl-glycyl. In still other aspects, the carrier is a polypeptide such as albumin, antitrypsin, macroglobulin, haptoglobin, caeruloplasm, transferrin, α- or β- lipoprotein, β- or γ- globulin or fibrinogen.

Approaches to designing peptide analogues, derivatives and mimetics are known in the art. For example, see Farmer, P. S. in Drug Design (E. J. Ariens, ed.) Academic Press, New York, 1980, vol. 10, pp. 119-143; Ball. J. B. and Alewood, P. F. (1990) J Mol. Recognition 3:55; Morgan, B. A. and Gainor, J. A. (1989) Ann. Rep. Med. Chem. 24:243; and Freidinger, R. M. (1989) Trends Pharmacol. Sci. 10:270. See also Sawyer, T. K. (1995) "Peptidomimetic Design and Chemical Approaches to Peptide Metabolism" in Taylor, M. D. and Amidon, G. L. (eds.) Peptide-Based Drug Design: Controlling Transport and Metabolism, Chapter 17; Smith, A. B. 3rd, et al. (1995) J. Am. Chem. Soc. 117:11113-11123; Smith, A. B. 3rd, et al. (1994) J. Am. Chem. Soc. 116:9947-9962; and Hirschman, R., et al. (1993) J. Am. Chem. Soc. 115:12550-12568.

Examples of peptide analogues, derivatives and peptidomimetics include peptides substituted with one or more benzodiazepine molecules (see e.g., James, G. L. et al. (1993) Science 260:1937-1942), peptides with methylated amide linkages and "retro-inverso" peptides (see U.S. Pat. No. 4,522,752 by Sisto).

Examples of peptide derivatives include peptides in which an amino acid side chain, the peptide backbone, or the amino- or carboxy-terminus has been derivatized (e.g., peptidic compounds with methylated amide linkages).

The term mimetic, and in particular, peptidomimetic, is intended to include isosteres. The term "isostere" refers to a chemical structure that can be substituted for a second chemical structure because the steric conformation of the first structure fits a binding site specific for the second structure. The term specifically includes peptide back-bone modifications (i.e., amide bond mimetics) well known to those skilled in the art. Such modifications include modifications of the amide nitrogen, the alpha-carbon, amide carbonyl, complete replacement of the amide bond, extensions, deletions or backbone crosslinks. Other examples ofisosteres include peptides substituted with one or more benzodiazepine molecules (see e.g., James, G. L. et al. (1993) Science 260:1937-1942)

Other possible modifications include an N-alkyl (or aryl) substitution ([CONR]), backbone crosslinking to construct lactams and other cyclic structures, substitution of all D-amino acids for all L-amino acids within the compound ("inverso" compounds) or retro-inverso amino acid incorporation ([NHCO]). By "inverso" is meant replacing L-amino acids of a sequence with D-amino acids, and by "retro-inverso" or "enantio-retro" is meant reversing the sequence of the amino acids ("retro") and replacing the L-amino acids with D-amino acids. For example, if the parent peptide is Thr-Ala-Tyr, the retro modified form is Tyr-Ala-Thr, the inverso form is thr-ala-tyr, and the retro-inverso form is tyr-ala-thr (lower case letters refer to D-amino acids). Compared to the parent peptide, a retro-inverso peptide has a reversed backbone while retaining substantially the original spatial conformation of the side chains, resulting in a retro-inverso isomer with a topology that closely resembles the parent peptide. See Goodman et al. "Perspectives in Peptide Chemistry" pp. 283-294 (1981). See also U.S. Pat. No. 4,522,752 by Sisto for further description of "retro-inverso" peptides.

A peptide can be attached to a compound of the invention through a functional group on the side chain of certain amino acids (e.g. serine) or other suitable functional groups. In an embodiment of the invention the carrier may comprise four or more amino acids with groups attached to three or more of the amino acids through functional groups on side chains. In another embodiment, the carrier is one amino acid, in particular a sulfonate derivative of an amino acid, for example cysteic acid.

"Disorders and/or diseases", "disorder(s)" and "disease(s)" are used interchangeably herein and include a condition characterized by abnormal protein folding or aggregation or abnormal amyloid formation, deposition, accumulation or persistence, or amyloid lipid interactions. In some aspects, the term includes conditions characterized by abnormal protein folding or aggregation or amyloid formation, deposition, accumulation or persistence. In particular aspects, the disease is a condition of the central or peripheral nervous system or systemic organ. In more particular aspects the terms include conditions associated with the formation, deposition, accumulation, or persistence of amyloid or amyloid fibrils, comprising an amyloid protein comprising or selected from the group consisting of Aβ amyloid, AA amyloid, AL amyloid, IAPP amyloid, PrP amyloid, α₂-microglobulin amyloid, transthyretin, prealbumin, and procalcitonin, especially Aβ amyloid and IAPP amyloid. A disorder and/or disease may be a condition where it is desirable to dissociate abnormally aggregated proteins and/or dissolve or disrupt pre-formed or pre-deposited amyloid or amyloid fibril.

In certain aspects of the invention the disease is amyloidosis. "Amyloidosis" refers to a diverse group of diseases of acquired or hereditary origin and characterized by the accumulation of one of several different types of protein fibrils with similar properties called amyloid. Amyloid can accumulate in a single organ or be dispersed throughout the body. The disease can cause serious problems in the affected areas, which may include the heart, brain, kidneys and digestive tract. The fibrillar composition of amyloid deposits is an identifying characteristic for various amyloid diseases. Intracerebral and cerebrovascular deposits composed primarily of fibrils of beta amyloid peptide (β-AP) are characteristic of Alzheimer's disease (both familial and sporadic forms); islet amyloid protein peptide (IAPP; amylin) is characteristic of the fibrils in pancreatic islet cell amyloid deposits associated with type II diabetes; and, β-2-microglobulin is a major component of amyloid deposits which form as a consequence of long term hemodialysis treatment. Prion-associated diseases, such as Creutzfeld-Jacob disease, scrapie, bovine spongiform encephalitis, and the like are characterized by the accumulation of a protease-resistant form of a prion protein (designated as AScr ro PrP-27).

Certain disorders are considered to be primary amyloidoses, in which there is no evidence for preexisting or coexisting disease. Primary amyloidoses are typically characterized by the presence of "amyloid light chain-type" (AL-type) protein fibrils. In secondary amyloidosis there is an underlying chronic inflammatory or infectious disease state (e.g., rheumatoid arthritis, juvenile chronic arthritis, ankylosing spondylitis, psoriasis, Reiter's syndrome, Adult Still's disease, Behcet's Syndrome, Crohn's disease, chronic microbial infections such as osteomyelitis, tuberculosis, and leprosy, malignant neoplasms such as Hodgkin's lymphoma, renal carcinoma, carcinomas of the gut, lung, and urogenital tract, basel cell carcinoma, and hairy cell carcinoma). Secondary amyloidosis is characterized by deposition of AA type fibrils derived from serum amyloid A protein (ApoSSA). Heredofamilial amyloidoses may have associated neuropathic, renal, or cardiovascular deposits of the ATTR transthyretin type, and they include other syndromes having different amyloid components (e.g., familial Mediterranean fever which is characterized by AA fibrils). Other forms of amyloidosis include local forms, characterized by focal, often tumor-like deposits that occur in isolated organs. In addition, amyloidoses are associated with aging, and are commonly characterized by plaque formation in the heart or brain. Amyloidoses includes systemic diseases such as adult-onset disabetes, complications from long-term hemodialysis and consequences of chronic inflammation or plasma cell dyscrasias.

Amyloid diseases that can be treated and/or prevented using the compounds, compositions and methods of the invention include without limitation, Alzheimer's disease, Down's syndrome, dementia pugilistica, multiple system atrophy, inclusion body myositosis, hereditary cerebral hemorrhage with amyloidosis of the Dutch type, Nieman-Pick disease type C, cerebral β-amyloid angiopathy, dementia associated with cortical basal degeneration, the amyloidosis of type 2 diabetes, the amyloidosis of chronic inflammation, the amyloidosis of malignancy and Familial Mediterranean Fever, the amyloidosis of multiple myeloma and B-cell dyscrasias, nephropathy with urticaria and deafness (Muckle - Wells syndrome), amyloidosis associated with systemic inflammatory diseases, idiopathic primary amyloidosis associated with myeloma or macroglobulinemia; amyloidosis associated with immunocyte dyscrasia; monoclonal gammopathy; occult dyscrasia; local nodular amyloidosis associated with chronic inflammatory diseases; amyloidosis associated with several immunocyte dyscrasias; familial amyloid polyneuropathy; hereditary cerebral hemorrhage with amyloidosis Alzheimer's disease and other neurodegenerative diseases, amyloidosis associated with chronic hemodialysis, diabetes type II, insulinoma, the amyloidosis of the prion diseases, (transmissible spongiform encephalopathies prion diseases), Creutzfeldt-Jakob disease, Gerstmann-Straussler syndrome, Kuru, and scrapie, the amyloidosis associated with carpal tunnel syndrome, senile cardiac amyloidosis, familial amyloidotic polyneuropathy, and the amyloidosis associated with endocrine tumors, especially Alzheimer's disease and type 2 diabetes.

In aspects of the invention, disorders and/or diseases include conditions associated with the formation, deposition, accumulation, or persistence of amyloid fibrils, especially the fibrils of an amyloid protein selected from the group consisting of Aβ amyloid, AA amyloid, AL amyloid, IAPP amyloid, PrP amyloid, α₂-microglobulin amyloid, transthyretin, prealbumin, and procalcitonin, especially Aβ amyloid and IAPP amyloid. Examples of such diseases include Alzheimer's disease, Down's syndrome, dementia pugilistica, multiple system atrophy, inclusion body myositosis, hereditary cerebral hemorrhage with amyloidosis ofthe Dutch type, Nieman-Pick disease type C, cerebral β-amyloid angiopathy, dementia associated with cortical basal degeneration, the amyloidosis of type 2 diabetes, the amyloidosis of chronic inflammation, the amyloidosis of malignancy and Familial Mediterranean Fever, the amyloidosis of multiple myeloma and B-cell dyscrasias, the amyloidosis of the prion diseases, Creutzfeldt-Jakob disease, Gerstmann-Straussler syndrome, kuru, and scrapie, the amyloidosis associated with carpal tunnel syndrome, senile cardiac amyloidosis, familial amyloidotic polyneuropathy, and the amyloidosis associated with endocrine tumors, especially Alzheimer's disease and type 2 diabetes.

In other aspects of the invention, disorders and/or diseases that can be treated and/or prevented using the compounds, compositions and methods of the invention include conditions of the central or peripheral nervous system or a systemic organ that result in the deposition of proteins, protein fragments, and peptides in beta-pleated sheets, fibrils, and/or aggregates or oligomers. In particular the disease is Alzheimer's disease, presenile and senile forms; amyloid angiopathy; mild cognitive impairment; Alzheimer's disease-related dementia (e.g., vascular or Alzheimer dementia); tauopathy (e.g., argyrophilic grain dementia, corticobasal degeneration, dementia pugilistica, diffuse neurofibrillary tangles with calcification, frontotemporal dementia with parkinsonism, Prion-related disease, Hallervorden-Spatz disease, myotonic dystrophy, Niemann-Pick disease type C, non-Guamanian Motor Neuron disease with neurofibrillary tangles, Pick's disease, postencephalitic parkinsonism, cerebral amyloid angiopathy, progressive subcortical gliosis, progressive supranuclear palsy, subacute sclerosing panencephalitis, and tangle only dementia), alpha-synucleinopathy (e.g., dementia with Lewy bodies, multiple system atrophy with glial cytoplasmic inclusions, Shy-Drager syndrome, spinocerebellar ataxia (e.g., DRPLA or Machado-Joseph Disease); striatonigral degeneration, olivopontocerebellar atrophy, neurodegeneration with brain iron accumulation type I, olfactory dysfunction, and amyotrophic lateral sclerosis); Parkinson's disease (e.g., familial or non-familial); Amyotrophic Lateral Sclerosis; Spastic paraplegia (e.g., associated with defective function of chaperones and/or triple A proteins); Huntington's Disease, spinocerebellar ataxia, Freidrich's Ataxia; neurodegenerative diseases associated with intracellular and/or intraneuronal aggregates of proteins with polyglutamine, polyalanine or other repeats arising from pathological expansions of tri- or tetra-nucleotide elements within corresponding genes; cerebrovascular diseases; Down's syndrome; head trauma with post-traumatic accumulation of amyloid beta peptide; Prion related disease (Creutzfeldt-Jakob disease, Gerstmann-Straussler-Scheinker disease, and variant Creutzfeldt-Jakob disease); Familial British Dementia; Familial Danish Dementia; Presenile Dementia with Spastic Ataxia; Cerebral Amyloid Angiopathy, British Type; Presenile Dementia With Spastic Ataxia Cerebral Amyloid Angiopathy, Danish Type; Familial encephalopathy with neuroserpin inclusion bodies (FENIB); Amyloid Polyneuropathy (e.g., senile amyloid polyneuropathy or systemic Amyloidosis); Inclusion Body myositis due to amyloid beta peptide; Familial and Finnish Type Amyloidosis; Systemic amyloidosis associated with multiple myeloma; Familial Mediterranean Fever; chronic infections and inflammations; and Type II Diabetes Mellitus associated with islet amyloid polypeptide (IAPP).

In aspects of the invention, in particular combination therapies, the disorder and/or disease is a neuronal disorder (e.g., Alzheimer's disease, Down Syndrome, Parkinson disease, Chorea Huntington, pathogenic psychotic conditions, schizophrenia, impaired food intake, sleep-wakefulness, impaired homeostatic regulation of energy metabolism, impaired autonomic function, impaired hormonal balance, impaired regulation, body fluids, hypertension, fever, sleep dysregulation, anorexia, anxiety related disorders including depression, seizures including epilepsy, drug withdrawal and alcoholism, neurodegenerative disorders including cognitive dysfunction and dementia).

The compounds of the invention may also act to inhibit or prevent α-synuclein/NAC fibril formation, inhibit or prevent α-synuclein/NAC fibril growth, and/or cause disassembly, disruption, and/or disaggregation of preformed α-synuclein/NAC fibrils and α-synuclein/NAC-associated protein deposits. Examples of synuclein diseases or synucleinopathies suitable for treatment with a compound or composition of the invention are diseases associated with the formation, deposition, accumulation, or persistence of synuclein fibrils, especially α-synuclein fibrils, including without limitation Parkinson's disease, familial Parkinson's disease, Lewy body disease, the Lewy body variant of Alzheimer's disease, dementia with Lewy bodies, multiple system atrophy, olivopontocerebellar atrophy, neurodegeneration with brain iron accumulation type I, olfactory dysfunction, and the Parkinsonism-dementia complex of Guam.

In aspects of the invention, the disease is a Motor Neuron Disease associated with filaments and aggregates of neurofilaments and/or superoxide dismutase proteins, the Spastic paraplegia associated with defective function of chaperones and/or triple A proteins, or a spinocerebellar ataxia such as DRPLA or Machado-Joseph Disease.

In other aspects of the invention, the disease is a Prion Disease including Creutzfeldt-Jakob disease, Gerstmann-Strausller-Scheinfer disease, and variant Creutzfeldt-Jakob disease and a Amyloid Polyneuropathy including senile amyloid polyneuropathy or systemic amyloidosis.

In embodiments of the invention, the disease is Alzheimer's disease or Parkinson's disease including familial and non-familial types.

In certain aspects of the invention, the disease may be characterized by an inflammatory process due to the presence of macrophages by an amyloidogenic protein or peptide. A method of the invention may involve inhibiting macrophage activation and/or inhibiting an inflammatory process. A method may comprise decreasing, slowing, ameliorating, or reversing the course or degree of macrophage invasion or inflammation in a patient.

A disease may be a condition that is associated with a molecular interaction that can be disrupted or dissociated with a compound of the invention. "A molecular interaction that can be disrupted or dissociated with a compound of the invention" includes an interaction comprising an amyloid protein and a protein or glycoprotein. An interaction comprising an amyloid protein includes an amyloid protein-amyloid protein interaction, amyloid-proteoglycan interaction, amyloid-proteoglycan/glycosaminoglycan (GAG) interaction and/or amyloid protein-glycosaminoglycan interaction. An interacting protein may be a cell surface, secreted or extracellular protein.

A disease that may be treated or prevented using a compound or composition ofthe invention includes a disease that would benefit from the disruption or dissolution of a molecular interaction comprising an amyloid protein and an interacting compound including a protein or glycoprotein. Examples of diseases that may be treated or prevented using a compound or composition of the invention include infectious diseases caused by bacteria, viruses, prions and fungi. Examples of such disorders and/or diseases are those associated with pathogens including *Herpes simplex* virus, *Pseudorabies* virus, human cytomegalovirus, human immunodeficiency virus, *Bordetellapertussis, Chlamydia trachomatis, Haemophilus influenzae, Helicobacter pylori, Borrelia burgdorferi, Neisseria gonorrhoeae, Mycobacterium tuberculosis, Staphylococcus aureus, Streptococcus mutans, Streptococcus suis, Plasmodium falciparum, Leishmania amazonensi, Trypanozoma cruzi, Listeria monocytogenes, Mycoplasma pneumoniae,* enterotoxigenic *E. coli,* uropathogenic *E.coli,* and *Pseudomonas aeruginosa*.

### Compositions

A scyllo-inositol compound may be formulated into a pharmaceutical composition or dietary supplement for administration to a subject. Pharmaceutical compositions of the present invention or fractions thereof typically comprise suitable pharmaceutically acceptable carriers, excipients, and vehicles selected based on the intended form of administration, and consistent with conventional pharmaceutical practices. Particular compositions of the invention can contain a scyllo-inositol compound that is pure or substantially pure.

Suitable pharmaceutical carriers, excipients, and vehicles are described in the standard text, Remington: The Science and Practice of Pharmacy, 21st Edition. University of the Sciences in Philadelphia (Editor), Mack Publishing Company. By way of example, for oral administration in the form of a capsule or tablet, the active components can be combined with an oral, non-toxic pharmaceutically acceptable inert carrier such as lactose, starch, sucrose, methyl cellulose, magnesium stearate, glucose, calcium sulfate, dicalcium phosphate, mannitol, sorbital, and the like. For oral administration in a liquid form, the drug components may be combined with any oral, non-toxic, pharmaceutically acceptable inert carrier such as ethanol, glycerol, water, and the like. Suitable binders (e.g. gelatin, starch, corn sweeteners, natural sugars including glucose; natural and synthetic gums, and waxes), lubricants (e.g. sodium oleate, sodium stearate, magnesium stearate, sodium benzoate, sodium acetate, and sodium chloride), disintegrating agents (e.g. starch, methyl cellulose, agar, bentonite, and xanthan gum), flavoring agents, and coloring agents may also be combined in the compositions or components thereof. Compositions as described herein can further comprise wetting or emulsifying agents, or pH buffering agents.

The invention provides commercially available formulations including without limitation pills, tablets, caplets, soft and hard gelatin capsules, lozenges, sachets, cachets, vegicaps, liquid drops, elixirs, suspensions, emulsions, solutions, syrups, aerosols (as a solid or in a liquid medium) suppositories, sterile injectable solutions, and/or sterile packaged powders, which contain a scyllo-inositol compound, in particular a pure or substantially pure scyllo-compound.

A composition can be a liquid solution, suspension, emulsion, tablet, pill, capsule, sustained release formulation, or powder. The compositions can be formulated as a suppository, with traditional binders and carriers such as triglycerides. Oral formulations can include standard carriers such as pharmaceutical grades of mannitol, lactose, starch, magnesium stearate, sodium saccharine, cellulose, magnesium carbonate, etc. Various delivery systems are known and can be used to administer a composition of the invention, e.g. encapsulation in liposomes, microparticles, microcapsules, and the like.

In aspects of the invention, a pharmaceutical composition is provided for oral administration of one or more scyllo-inositol compound for treatment of a disease and/or disorder. In a particular aspect, a stable oral pharmaceutical composition for treatment of a disease and/or disorder characterized by abnormal protein folding and/or aggregation, and/or amyloid formation, deposition, accumulation, or persistence (e.g., Alzheimer's disease) is provided comprising a substantially pure scyllo-inositol compound of the formula Ia or Ib.

Formulations for parenteral administration may include aqueous solutions, syrups, aqueous or oil suspensions and emulsions with edible oil such as cottonseed oil, coconut oil, almond oil, or peanut oil. Dispersing or suspending agents that can be used for aqueous suspensions include synthetic or natural gums, such as tragacanth, alginate, acacia, dextran, sodium carboxymethylcellulose, gelatin, methylcellulose, and polyvinylpyrrolidone.

Compositions for parenteral administration may include sterile aqueous or non-aqueous solvents, such as water, isotonic saline, isotonic glucose solution, buffer solution, or other solvents conveniently used for parenteral administration of therapeutically active agents. A composition intended for parenteral administration may also include conventional additives such as stabilizers, buffers, or preservatives, e.g. antioxidants such as methylhydroxybenzoate or similar additives.

Compositions of the invention can be formulated as pharmaceutically acceptable salts as described herein.

In aspects of the invention, the compositions include without limitation at least one buffering agent or solution. Examples of buffering agents include, but are not limited to hydrochloric, hydrobromic, hydroiodic, sulfuric, phosphoric, formic, acetic, propionic, succinic, glycolic, glucoronic, maleic, furoic, citric, glutamic, benzoic, anthranilic, salicylic, phenylacetic, mandelic, embonic, pamoic, methanesulfonic, ethanesulfonic, pantothenic, benzenesulfonic, stearic, sulfanilic, algenic, galacturonic acid and mixtures thereof. Additional agents may also be included such as one or more of pregelatinized maize starch, polyvinyl pyrrolidone, hydroxypropyl methylcellulose, lactose, microcrystalline cellulose, calcium hydrogen phosphate, magnesium stearate, talc, silica, potato starch, sodium starch glycolate, sodium lauryl sulfate, sorbitol syrup, cellulose derivatives, hydrogenated edible fats, lecithin, acacia, almond oil, oily esters, ethyl alcohol, fractionated vegetable oils, methyl, propyl-p-hydroxybenzoates, sorbic acid and mixtures thereof. A buffering agent may additionally comprise one or more of dichlorodifluoromethane, trichloro fluoromethane, dichlorotetra fluoroethane, carbon dioxide, poly (N-vinyl pyrrolidone), poly (methylmethacrylate), polyactide, polyglycolide and mixtures thereof. In an embodiment, a buffering agent can be formulated as at least one medium including without limitation a suspension, solution, or emulsion. In other embodiments, a buffering agent may additionally comprise a formulatory agent including without limitation a pharmaceutically acceptable carrier, excipient, suspending agent, stabilizing agent or dispersing agent.

A composition of the invention may be sterilized by, for example, filtration through a bacteria retaining filter, addition of sterilizing agents to the composition, irradiation of the composition, or heating the composition. Alternatively, the compounds or compositions of the present invention may be provided as sterile solid preparations e.g. lyophilized powder, which are readily dissolved in sterile solvent immediately prior to use.

After pharmaceutical compositions have been prepared, they can be placed in an appropriate container and labeled for treatment of an indicated condition. For administration of a composition of the invention, such labeling would include amount, frequency, and method of administration.

A scyllo-inositol compound may be in a form suitable for administration as a dietary supplement. A supplement of the invention may optionally include inactive ingredients such as diluents or fillers, viscosity-modifying agents, preservatives, flavorings, colorants, or other additives conventional in the art. By way of example only, conventional ingredients such as beeswax, lecithin, gelatin, glycerin, caramel, and carmine may be included.

A dietary supplement composition of the invention may optionally comprise a second active ingredient. In an embodiment, the second active ingredient is pinitol or an active derivative or metabolite thereof. Pinitol can be produced from plant sources, including without limitation alfalfa, Bougainvillea leaves, chick peas, pine trees and soy beans. Pinitol is also commercially available, for example Inzitol™ (Humanetics Corporation, Min). Examples of derivatives and metabolites of pinitol include without limitation pinitol glycosides, pinitol phospholipids, esterified pinitol, lipid-bound pinitol, pinitol phosphates, pinitol phytates, and hydrolyzed pinitol such as d-chiro-inositol.

A dietary supplement may be provided as a liquid dietary supplement (e.g., a dispensable liquid) or alternatively the compositions may be formulated as granules, capsules or suppositories. The liquid supplement may include a number of suitable carriers and additives including water, glycols, oils, alcohols, flavoring agents, preservatives, coloring agents and the like. In capsule, granule or suppository form, the compositions of the present invention are formulated in admixture with a pharmaceutically acceptable carrier.

In an aspect, a dietary supplement of the present invention is formulated as a beverage, but may be formulated in granule, capsule or suppository form.

A supplement may be presented in the form of a softgel which is prepared using conventional methods. A softgel typically includes a layer of gelatin encapsulating a small quantity of the supplement. A supplement may also be in the form of a liquid-filled and sealed gelatin capsule, which may be made using conventional methods.

To prepare a dietary supplement composition of the present invention in capsule, granule or suppository form, one or more compositions of the present invention may be intimately admixed with a pharmaceutically acceptable carrier according to conventional formulation techniques. For solid oral preparations such as capsules and granules, suitable carriers and additives such as starches, sugars, diluents, granulating agents, lubricants, binders, disintegrating agents and the like may be included.

According to another aspect of the invention, a kit is provided. In an aspect, the kit comprises a compound or a pharmaceutical composition of the invention. The kit can be a package which houses a container which contains a composition of the invention and also houses instructions for administering the composition to a subject.

In embodiments of the invention, a pharmaceutical pack or kit is provided comprising one or more containers filled with one or more of the ingredients of a pharmaceutical composition of the invention to provide a beneficial effect, in particular a sustained beneficial effect. Associated with such container(s) can be various written materials such as instructions for use, or a notice in the form prescribed by a governmental agency regulating the labeling, manufacture, use or sale of pharmaceuticals or biological products, which notice reflects approval by the agency of manufacture, use, or sale for human administration.

### Applications

The invention is related to compositions and methods that utilize one or more scyllo-inositol compound to provide beneficial effects. In particular, the invention contemplates the use of a composition of the invention for treating a disorder and/or disease, in particular preventing, and/or ameliorating disease severity, disease symptoms, and/or periodicity of recurrence of a disorder and/or disease disclosed herein. The invention also contemplates preventing and/or treating in mammals, disorders and/or diseases using the compositions or treatments of the invention. The present invention in embodiments may provide a composition comprising a compound that provides beneficial effects including greater solubility, stability, efficacy, potency, and/or utility, in particular greater solubility and stability.

In an aspect, the invention provides a method of improving memory of a healthy subject or the memory of a subject with age impaired memory by administering an effective amount of one or more scyllo-inositol compound, or a composition comprising one or more scyllo-inositol compound, and a pharmaceutically acceptable carrier, excipient, or vehicle.

In another aspect, the present invention further relates to a method for improving memory, especially short-term memory and other mental dysfunction associated with the aging process comprising administering an effective amount of one or more scyllo-inositol compound, or a pharmaceutically acceptable salt thereof, or a composition comprising one or more scyllo-inositol compound, and a pharmaceutically acceptable carrier, excipient, or vehicle.

In an embodiment, a method is provided for treating a mammal in need of improved memory, wherein said mammal has no diagnosed disease, disorder, infirmity or ailment known to impair or otherwise diminish memory, comprising the step of administering to the mammal an effective memory-improving amount of one or more scyllo-inositol compound, a pharmaceutically acceptable salt thereof, or a dietary supplement comprising one or more scyllo-inositol compound or a nutraceutically acceptable derivative thereof.

In another aspect of the invention, a method is provided for treating in a subject a condition of the central or peripheral nervous system or systemic organ associated with a disorder in protein folding or aggregation, or amyloid formation, deposition, accumulation, or persistence, comprising administering to the subject a therapeutically effective amount of one or more scyllo-inositol compound, or a pharmaceutically acceptable salt thereof, or a composition comprising one or more scyllo-inositol compound and a pharmaceutically acceptable carrier, excipient, or vehicle.

In a further aspect, the invention provides a method involving administering to a subject a therapeutic compound of one or more scyllo-inositol compound, or pharmaceutically acceptable salts thereof, or a composition comprising one or more scyllo-inositol compound, and a pharmaceutically acceptable carrier, excipient, or vehicle which inhibit amyloid formation, deposition, accumulation and/or persistence, and/or which cause dissolution/disruption of pre-existing amyloid. Thus, the compounds and compositions of the invention may be used for inhibiting amyloidosis in disorders in which amyloid deposition occurs.

In another aspect, the invention provides a method for treating in a subject a condition associated with an amyloid interaction that can be disrupted or dissociated with a compound of the invention comprising administering to the subject a therapeutically effective amount of one or more scyllo-inositol compound, a pharmaceutically acceptable salt thereof, or a composition comprising one or more scyllo-inositol compound and a pharmaceutically acceptable carrier, excipient, or vehicle.

In an aspect, the invention provides a method for preventing, reversing, reducing or inhibiting amyloid protein assembly, enhancing clearance of amyloid deposits, or slowing deposition of amyloid deposits in a subject comprising administering a therapeutically effective amount of one or more scyllo-inositol compound, a pharmaceutically acceptable salt thereof, or a composition comprising one or more scyllo-inositol compound, and a pharmaceutically acceptable carrier, excipient, or vehicle.

In an aspect, the invention provides a method for preventing, reversing, reducing or inhibiting amyloid fibril formation, organ specific dysfunction (e.g., neurodegeneration), or cellular toxicity in a subject comprising administering to the subject a therapeutically effective amount of one or more scyllo-inositol compound, a pharmaceutically acceptable salt thereof, or a composition comprising one or more scyllo-inositol compound, and a pharmaceutically acceptable carrier, excipient, or vehicle.

In another aspect, the invention provides a method of preventing or reversing conformationally altered protein assembly or aggregation in an animal that includes introducing one or more scyllo-inositol compound including, its analogs, or derivatives to the conformationally altered protein.

In a further aspect of the invention, a method of preventing or reversing conformationally altered protein assembly or aggregation in an animal is provided that includes introducing one or more scyllo-inositol compound to the conformationally altered protein.

In a still further aspect of the invention, a method of treating conformationally altered protein assembly or aggregation in an animal is provided that includes administering a therapeutically effective amount of compositions of the invention.

In an aspect, the invention provides a method for increasing or maintaining synaptic function in a subject comprising administering a therapeutically effective amount of one or more scyllo-inositol compound, a pharmaceutically acceptable salt thereof, or a composition comprising one or more scyllo-inositol compound, and a pharmaceutically acceptable carrier, excipient, or vehicle.

The invention has particular applications in treating a disorder and/or disease characterized by amyloid deposition, in particular an amyloidoses, more particularly Alzheimer's disease. Thus, the invention relates to a method of treatment comprising administering a therapeutically effective amount of one or more scyllo-inositol compound, a pharmaceutically acceptable salt thereof, or a composition comprising a scyllo-inositol compound and a pharmaceutically acceptable carrier, excipient, or vehicle, which upon administration to a subject with symptoms of a disease characterized by amyloid deposition, more particularly Alzheimer's disease, produces beneficial effects, preferably sustained beneficial effects. In an embodiment, beneficial effects are evidenced by one or more of the following: disruption of aggregated Aβ or Aβ oligomers, increased or restored long term potentiation, and/or maintenance of or increased synaptic function, and/or, reduced cerebral accumulation of Aβ, deposition of cerebral amyloid plaques, soluble Aβ oligomers in the brain, glial activity, inflammation, and/or cognitive decline.

In an aspect, the invention provides a method for amelioriating progression of a disorder and/or disease or obtaining a less severe stage of a disease in a subject suffering from such disease (e.g. Alzheimer's disease) comprising administering a therapeutically effective amount of one or more scyllo-inositol compound, a pharmaceutically acceptable salt thereof, or a composition comprising one or more scyllo-inositol compound, and a pharmaceutically acceptable carrier, excipient, or vehicle.

In another aspect, the invention relates to a method of delaying the progression of a disorder and/or disease (e.g. Alzheimer's disease) comprising administering a therapeutically effective amount of one or more scyllo-inositol compound, a pharmaceutically acceptable salt thereof, or a composition comprising one or more scyllo-inositol compound, and a pharmaceutically acceptable carrier, excipient, or vehicle.

In a further aspect, the invention relates to a method of increasing survival of a subject suffering from a disorder and/or disease comprising administering a therapeutically effective amount of one or more scyllo-inositol compound, a pharmaceutically acceptable salt thereof, or a composition comprising one or more scyllo-inositol compound, and a pharmaceutically acceptable carrier, excipient, or vehicle.

In an embodiment, the invention relates to a method of improving the lifespan of a subject suffering from a disorder and/or disease (e.g., Alzheimer's disease) comprising administering a therapeutically effective amount of one or more scyllo-inositol compound, a pharmaceutically acceptable salt thereof, or a composition comprising one or more scyllo-inositol compound, and a pharmaceutically acceptable carrier, excipient, or vehicle.

In an aspect the invention provides a method for treating mild cognitive impairment (MCI) comprising administering a therapeutically effective amount of one or more scyllo-inositol compound, a pharmaceutically acceptable salt thereof, or a composition comprising one or more scyllo-inositol compound, and a pharmaceutically acceptable carrier, excipient, or vehicle.

In an embodiment, the invention provides a method of reducing or reversing amyloid deposition and neuropathology after the onset of cognitive deficits and amyloid plaque neuropathology in a subject comprising administering to the subject a therapeutically effective amount of one or more scyllo-inositol compound, a pharmaceutically acceptable salt thereof, or a composition comprising one or more scyllo-inositol compound and a pharmaceutically acceptable carrier, excipient, or vehicle.

In another embodiment, the invention provides a method of reducing or reversing amyloid deposition and neuropathology after the onset of cognitive deficits and amyloid plaque neuropathology in a subject comprising administering to the subject an amount of one or more scyllo-inositol compound, a pharmaceutically acceptable salt thereof, or a composition comprising one or more scyllo-inositol compound and a pharmaceutically acceptable carrier, excipient, or vehicle effective to reduce or reverse amyloid deposition and neuropathology after the onset of cognitive deficits and amyloid plaque neuropathology.

Aspects of the invention provide improved methods and compositions for use of one or more scyllo-inositol compound for sustained treatment of a disorder and/or disease (e.g., Alzheimer's disease). The present invention in an embodiment provides a composition comprising one or more scyllo-inositol compound that achieves greater efficacy, potency, and utility. For example, the greater efficacy can be shown by improving or reversing cognitive decline and/or survival in Alzheimer's disease with treatment resulting in sustained improvement and/or increased survival after ceasing treatment.

In an aspect of the invention a compound of the formula Ia or Ib is utilized in the treatment of Alzheimer's disease. Thus, Alzheimer's disease may be treated by administering a therapeutically effective amount of a compound of the formula Ia or formula Ib. Such treatment may be effective for retarding the degenerative effects of Alzheimer's disease, including specifically, but not exclusively, deterioration of the central nervous system, loss of mental facilities, loss of short term memory, and disorientation.

In an embodiment, where the disease is Alzheimer's disease, beneficial effects of a compound or composition or treatment of the invention can manifest as at least one, two, three, four, five, six, seven, eight, nine, ten, twelve, thirteen, fourteen, fifteen, or all of the following, in particular five or ten or more, more particularly fifteen or more of the following:
a) An increase or restoration of long term potentiation relative to the level in the absence of a compound disclosed herein after administration to a subject with symptoms of Alzheimer's disease. In aspects of the invention the compounds induce at least about a 0.05%, 0.1%, 0.5%, 1%, 2%, 5%, 10%, 15%, 20%, 30%, 33%, 35%, 40%, 45%, 50%, 60%, 70%, 80%, 90%, 95%, or 99% increase in long term potentiation in a subject.
b) An increase or maintenance of synaptic function relative to the level of synaptic function in the absence of a compound disclosed herein after administration to a subject with symptoms of Alzheimer's disease. In aspects of the invention the compounds induce at least about a 0.05%, 0.1%, 0.5%, 1%, 2%, 5%, 10%, 15%, 20%, 30%, 33%, 35%, 40%, 45%, 50%, 60%, 70%, 80%, 90%, 95%, 99%, 100%, 125%, 150%, 175% or 200% increase in synaptic function in a subject.
c) An increase in synaptophysin. In aspects of the invention there is at least about a 2%, 5%, 10%, 15%, 20%, 30%, 40%, 50%, 60%, 70%, 80%, 90%, 95%, 99%, 100%, 125%, 150%, 175% or 200% increase in synaptophysin.
d) An increase in synaptophysin reactive boutons and cell bodies. In aspects of the invention there is at least about a 2%, 5%, 10%, 15%, 20%, 30%, 40%, 50%, 60%, 70%, 80%, 90%, 95%, 99%, 100%, 125%, 150%, 175% or 200%, more particularly about a 100-150% or 140-150%, increase in synaptophysin reactive boutons and cell bodies,.
e) A reduction or an absence of symptoms of inflammation, in particular a Aβ-induced inflammatory response, after administration to a subject with symptoms of Alzheimer's disease.
f) A reduction in cerebral accumulation of amyloid β relative to the levels measured in the absence of a scyllo-inositol compound in subjects with symptoms of Alzheimer's disease. In aspects of the invention, the compounds induce at least about a 2%, 5%, 10%, 15%, 20%, 30%, 40%, 50%, 60%, 70%, 80%, or 90% decrease in cerebral accumulation of amyloid β.
g) A reduction in deposition of cerebral amyloid plaques, relative to the levels measured in the absence of a scyllo-inositol compound in subjects with symptoms of Alzheimer's disease. In aspects of the invention, the compounds induce at least about a 2%, 5%, 10%, 15%, 20%, 30%, 40%, 50%, 60%, 70%, 80%, or 90% decrease in deposition of cerebral amyloid plaques.
h) A reduction in plaque number. In aspects of the invention, the compounds induce at least about a 2%, 5%, 10%, 15%, 20%, 30%, 40%, 50%, 60%, 70%, 80%, or 90% reduction in plaque number. In particular aspects the compounds induce a 5-15% or 10-15% reduction in plaque number.
i) A reduction in plaque size. In aspects of the invention, the compounds induce at least about a 2%, 5%, 10%, 15%, 20%, 30%, 40%, 50%, 60%, 70%, 80%, or 90% reduction in plaque size. In particular aspects the compounds induce a 5-15% or 10-15% reduction in plaque size.
j) A reduction in percent area of the brain covered in plaques. In aspects of the invention, the compounds induce at least about a 2%, 5%, 10%, 15%, 20%, 30%, 40%, 50%, 60%, 70%, 80%, or 90% reduction in percent area of the brain covered in plaques. In particular aspects the compounds induce a 5-15% or 10-15% reduction in percent area of the brain covered in plaques.
k) A reduction in soluble Aβ oligomers in the brain, relative to the levels measured in the absence of a compound disclosed herein in subjects with symptoms of Alzheimer's disease. In aspects of the invention, the compounds induce at least about a 2%, 5%, 10%, 15%, 20%, 30%, 40%, 50%, 60%, 70%, 80%, or 90% decrease in soluble Aβ oligomers.
l) A reduction in brain levels of Aβ40. In aspects of the invention, the compounds induce at least about a 2%, 5%, 10%, 15%, 20%, 30%, 40%, 50%, 60%, 70%, 80%, or 90% reduction in Aβ40. In particular aspects the compounds induce a 10-50%, 20-45%, or 25-35% reduction in brain levels of Aβ40.
m) A reduction in brain levels of Aβ42. In aspects of the invention, the compounds induce at least about a 2%, 5%, 10%, 15%, 20%, 30%, 40%, 50%, 60%, 70%, 80%, or 90% reduction in Aβ42. In particular aspects the compounds induce a 10-50%, 15-40%, or 20-25% reduction in brain levels of Aβ42.
n) A reduction in glial activity in the brain, relative to the levels measured in the absence of a compound disclosed herein in subjects with symptoms of Alzheimer's disease. Preferably, the compounds induce at least about a 2%, 5%, 10%, 15%, 20%, 30%, 40%, 50%, 60%, 70%, 80%, or 90% decrease in glial activity
o) Maintenance of synaptic function at about normal for a prolonged period of time, in particular for at least 5 weeks, 6 weeks, 8 weeks, 10 weeks, 12 weeks, 14 weeks, 16 weeks, 20 weeks, 24 weeks, 30 weeks, 40 weeks, 52 weeks, or 78 weeks, more particularly, 2 to 4 weeks, 2 to 5 weeks, 3 to 5 weeks, 2 to 6 weeks, 2 to 8 weeks, 2 to 10 weeks, 2 to 12 weeks, 2 to 16 weeks, 2 to 20 weeks, 2 to 24 weeks, 2 weeks to 12 months, or 2 weeks to 24 months following treatment.
p) A reduction or slowing of the rate of disease progression in a subject with Alzheimer's disease. In particular a reduction or slowing of cognitive decline in a subject with Alzheimer's disease.
q) A reduction or slowing of cognitive deficits.
r) A reduction in or slowing of amyloid angiopathy.
s) A reduction in accelerated mortality.
t) An increase in survival in a subject with symptoms of Alzheimer's disease.

In aspects of the invention beneficial effects of a composition or treatment of the invention can manifest as (a) and (b); (a), (b) and (c); (a), (b), (e), (f) and (g); (a), (b), (e), (f) through (h); (a), (b), (e), (f) through (i); (a), (b), (e), (f) through (j); (a), (b), (e), (f) through (k); (a), (b), (e), (f) through (l); (a), (b), (e), (f) through (m); (a), (b), (e), (f) through (n); (a), (b), (e), (f) through (o); (a), (b), (e), (f) through (p); (a), (b), (e), (f) through (q); (a), (b), (e), (f) through (r); (a), (b), (e), (f) through (s); (a), (b), (e), (f) through (t); (a) through (d); (a) through (e); (a) through (f); (a) through (g); (a) through (h); (a) through (i); (a) through (j); (a) through (k); (a) through (l); (a) through (m); (a) through (n); (a) through (o); (a) through (p); (a) through (q); (a) through (r); (a) through (s); and (a) through (t).

Compounds, pharmaceutical compositions and methods of the invention can be selected that have statistically significant beneficial effects, in particular one or more of the effects of (a) through (t) above. Compounds, pharmaceutical compositions and methods of the invention can also be selected that have sustained beneficial effects, in particular statistically significant sustained beneficial effects. In an embodiment, a pharmaceutical composition is provided with statistically significant sustained beneficial effects, in particular sustained beneficial effects of one or more of (a) through (t) above, comprising a therapeutically effective amount of one or more scyllo-inositol compound. In aspects of the invention, one or more of the beneficial effects provide enhanced therapeutic effects compared with conventional therapies.

Greater efficacy and potency of a treatment of the invention in some aspects may improve the therapeutic ratio of treatment, reducing untoward side effects and toxicity. Selected methods of the invention may also improve long-standing Alzheimer's disease even when treatment is begun long after the appearance of symptoms. Prolonged efficacious treatment can be achieved in accordance with the invention following administration of a compound or composition of the invention.

In an aspect, the invention relates to a method for treating Alzheimer's disease comprising contacting Aβ, Aβ aggregates, or Aβ oligomers in particular Aβ40 or Aβ40 aggregates or oligomers and/or Aβ42 or Aβ42 aggregates or oligomers, in a subject with a therapeutically effective amount of one or more scyllo-inositol compound or a composition comprising a scyllo-inositol compound.

In another aspect, the invention provides a method for treating Alzheimer's disease by providing a composition comprising one or more scyllo-inositol compound in an amount sufficient to disrupt aggregated Aβ or Aβ oligomers for a prolonged period following administration.

In a further aspect, the invention provides a method for treating Alzheimer's disease in a patient in need thereof which includes administering to the individual a composition that provides one or more scyllo-inositol compound in a dose sufficient to increase or restore long term potentiation and/or maintain synaptic function. In another aspect, the invention provides a method for treating Alzheimer's disease comprising administering, preferably orally or systemically, an amount of a scyllo-inositol compound to a mammal, to reduce cerebral accumulation of Aβ, deposition of cerebral amyloid plaques, soluble Aβ oligomers in the brain, glial activity, and/or inflammation for a prolonged period following administration.

The invention in an embodiment provides a method for treating Alzheimer's disease, the method comprising administering to a mammal in need thereof a composition comprising one or more scyllo-inositol compound in an amount sufficient to reduce cognitive decline, especially for a prolonged period following administration, thereby treating the Alzheimer's disease.

The invention in an embodiment provides a method for treating Alzheimer's disease, the method comprising administering to a mammal in need thereof a composition comprising one or more scyllo-inositol compound in an amount sufficient to increase or maintain synaptic function, especially for a prolonged period following administration, thereby treating the Alzheimer's disease.

In another aspect, the invention provides a method for preventing and/or treating Alzheimer's disease, the method comprising administering to a mammal in need thereof a composition comprising one or more scyllo-inositol compound in an amount sufficient to disrupt aggregated Aβ or Aβ oligomers for a prolonged period following administration; and determining the amount of aggregated Aβ or Aβ oligomers, thereby treating the Alzheimer's disease. The amount of aggregated Aβ or Aβ oligomers may be measured using an antibody specific for Aβ or a scyllo-inositol labeled with a detectable substance.

The present invention also includes methods of using the compositions of the invention in combination treatments with one or more additional therapeutic agents including without limitation beta-secretase inhibitors, gamma-secretase inhibitors, epsilon-secretase inhibitors, other inhibitors of beta-sheet aggregation/fibrillogenesis/ADDL formation (e.g. Alzhemed), NMDA antagonists (e.g. memantine), non-steroidal anti-inflammatory compounds (e.g. Ibuprofen, Celebrex), anti-oxidants (e.g. Vitamin E), hormones (e.g. estrogens), nutrients and food supplements (e.g. Gingko biloba), statins and other cholesterol lowering drugs (e.g. Lovastatin and Simvastatin), acetylcholinesterase inhibitors (e.g. donezepil), muscarinic agonists (e.g. AF102B (Cevimeline, EVOXAC), AF150(S), and AF267B), anti-psychotics (e.g. haloperidol, clozapine, olanzapine), anti-depressants including tricyclics and serotonin reuptake inhibitors (e.g. Sertraline and Citalopram Hbr), statins and other cholesterol lowering drugs (e.g. Lovastatin and Simvastatin), immunotherapeutics and antibodies to Aβ (e.g. ELAN AN-1792), vaccines, inhibitors of kinases (CDK5, GSK3α, GSK3β) that phosphorylate TAU protein (e.g. Lithium chloride), inhibitors ofkinases that modulate Aβ production (GSK3α, GSK3β, Rho/ROCK kinases) (e.g. lithium Chloride and Ibuprofen), drugs that upregulate neprilysin (an enzyme which degrades Aβ); drugs that upregulate insulin degrading enzyme (an enzyme which degrades Aβ), agents that are used for the treatment of complications resulting from or associated with a disease, or general medications that treat or prevent side effects. The present invention also includes methods of using the compositions of the invention in combination treatments with one or more additional treatments including without limitation gene therapy and/or drug based approaches to upregulate neprilysin (an enzyme which degrades Aβ), gene therapy and/or drug based approaches to upregulate insulin degrading enzyme (an enzyme which degrades Aβ), or stem cell and other cell-based therapies.

Combinations of a scyllo-inositol compound and a therapeutic agent or treatment may be selected to provide unexpectedly additive effects or greater than additive effects i.e. synergistic effects. Other therapeutics and therapies may act via a different mechanism and may have additive/synergistic effects with the present invention

A composition or method (i.e., combination treatment) comprising one or more scyllo-inositol compound and a therapeutic agent employing different mechanisms to achieve maximum therapeutic efficacy, may improve tolerance to the therapy with a reduced risk of side effects that may result from higher doses or longer term monotherapies (i.e. therapies with each compound alone). A combination treatment may also permit the use of lower doses of each compound with reduced adverse toxic effects of each compound. A suboptimal dosage may provide an increased margin of safety, and may also reduce the cost of a drug necessary to achieve prophylaxis and therapy. In addition, a treatment utilizing a single combination dosage unit may provide increased convenience and may result in enhanced compliance. Other advantages of a combination therapy may include higher stability towards degradation and metabolism, longer duration of action, and/or longer duration of action or effectiveness at particularly low doses.

In an aspect, the invention contemplates the use of a composition comprising at least one scyllo-inositol compound for the preparation of a medicament in treating a disorder and/or disease. The invention also contemplates the use of a composition comprising at least one scyllo-inositol compound for the preparation of a medicament for preventing and/or treating disorders and/or diseases. The invention additionally provides uses of a pharmaceutical composition of the invention in the preparation of medicaments for the prevention and/or treatment of disorders and/or diseases. The medicaments provide beneficial effects, preferably sustained beneficial effects following treatment. The medicament may be in a form for consumption by a subject such as a pill, tablet, caplet, soft and hard gelatin capsule, lozenge, sachet, cachet, vegicap, liquid drop, elixir, suspension, emulsion, solution, syrup, aerosol (as a solid or in a liquid medium) suppository, sterile injectable solution, and/or sterile packaged powder for inhibition of amyloid formation, deposition, accumulation, and/or persistence, regardless of its clinical setting.

In an embodiment, the invention relates to the use of a therapeutically effective amount of at least one scyllo-inositol compound or a composition of the invention for preparation of a medicament for providing therapeutic effects, in particular beneficial effects, preferably sustained beneficial effects, in treating a disorder and/or disease.

In another embodiment the invention provides the use of one or more scyllo-inositol compound or composition of the invention for the preparation of a medicament for prolonged or sustained treatment of Alzheimer's disease.

In a further embodiment the invention provides the use of a scyllo-inositol compound for preparation of a pharmaceutical composition to be employed through oral administration for treatment of a disorder characterized by abnormal protein folding and/or aggregation, and/or amyloid formation, deposition, accumulation, or persistence.

Therapeutic efficacy and toxicity of compositions and methods of the invention may be determined by standard pharmaceutical procedures in cell cultures or with experimental animals such as by calculating a statistical parameter such as the ED₅₀ ( the dose that is therapeutically effective in 50% of the population) or LD₅₀ (the dose lethal to 50% of the population) statistics. The therapeutic index is the dose ratio of therapeutic to toxic effects and it can be expressed as the ED₅₀/LD₅₀ ratio. Pharmaceutical compositions which exhibit large therapeutic indices are preferred. One or more of the therapeutic effects, in particular beneficial effects disclosed herein, can be demonstrated in a subject or disease model. For example, beneficial effects may be demonstrated in a model described in the Examples herein, in particular beneficial effects may be demonstrated in a TgCRND8 mouse with symptoms of Alzheimer's disease.

The methods of the invention may further comprise measuring Aβ as a marker. In an aspect the invention relates to methods of assessing the efficacy of a treatment for a disease characterized by amyloid deposition, more particularly Alzheimer's disease in a subject comprising detecting Aβ40 and/or Aβ42 in a sample from the subject with a scyllo-inositol compound labelled with a detectable substance before treatment with an agent. An amount of Aβ40 and/or Aβ42 in the sample from the subject after treatment with the agent is compared to the baseline amount of Aβ40 and/or Aβ42. A reduction between the amount of Aβ40 and/or Aβ42 measured after the treatment compared to the baseline amount indicates a positive treatment outcome. The amount of Aβ40 and/or Aβ42 can be measured at increasing intervals following administration of the agent. A sustained reduction of Aβ40 and/or Aβ42 (e.g. sustained for more than 3, 6, 12, 18, or 24 months) can indicate that the agent provides sustained beneficial effects. The amount of Aβ40 and/or Aβ42 in a subject's sample can also be compared to a control value determined from a population of patients experiencing amelioriation of, or freedom from, symptoms of disease due to the treatment agent. A value in the subject at least equal to the control value indicates a positive response to the treatment.

### Administration

Scyllo-inositol compounds and compositions of the present invention can be administered by any means that produce contact of the active agent(s) with the agent's sites of action in the body of a subject or patient to produce a therapeutic effect, in particular a beneficial effect, in particular a sustained beneficial effect. The active ingredients can be administered simultaneously or sequentially and in any order at different points in time to provide the desired beneficial effects. A compound and composition of the invention can be formulated for sustained release, for delivery locally or systemically. It lies within the capability of a skilled physician or veterinarian to select a form and route of administration that optimizes the effects of the compositions and treatments of the present invention to provide therapeutic effects, in particular beneficial effects, more particularly sustained beneficial effects.

The compounds and compositions may be administered in oral dosage forms such as tablets, capsules (each of which includes sustained release or timed release formulations), pills, powders, granules, elixirs, tinctures, suspensions, syrups, and emulsions. They may also be administered in intravenous (bolus or infusion), intraperitoneal, subcutaneous, or intramuscular forms, all utilizing dosage forms well known to those of ordinary skill in the pharmaceutical arts. The compositions of the invention may be administered by intranasal route via topical use of suitable intranasal vehicles, or via a transdermal route, for example using conventional transdermal skin patches. A dosage protocol for administration using a transdermal delivery system may be continuous rather than intermittent throughout the dosage regimen. A sustained release formulation can also be used for the therapeutic agents.

In aspects of the invention the compounds and compositions are administered by peripheral administration, in particular by intravenous administration, intraperitoneal administration, subcutaneous administration, intramuscular administration, oral administration, topical administration, transmucosal administration, or pulmonary administration.

The dosage regimen of the invention will vary depending upon known factors such as the pharmacodynamic characteristics of the agents and their mode and route of administration; the species, age, sex, health, medical condition, and weight of the patient, the nature and extent of the symptoms, the kind of concurrent treatment, the frequency of treatment, the route of administration, the renal and hepatic function of the patient, and the desired effect.

An amount of a scyllo-inositol compound or composition comprising same which will be effective in the treatment of a particular disorder and/or disease to provide effects, in particular beneficial effects, more particularly sustained beneficial effects, will depend on the nature of the disorder and/or disease, and can be determined by standard clinical techniques. The precise dose to be employed in the formulation will also depend on the route of administration, and the seriousness of the disease, and should be decided according to the judgment of the practitioner and each patient's circumstances.

Suitable dosage ranges for administration are particularly selected to provide therapeutic effects, in particular beneficial effects, more particularly sustained beneficial effects. A dosage range is generally effective for triggering the desired biological responses. The dosage ranges are generally about .5 mg to about 2 g per kg, about 1 mg to about 1 g per kg, about 1 mg to about 200 mg per kg, about 1 mg to about 100 mg per kg, about 1 mg to about 50 mg per kg, about 10 mg to about 100 mg per kg, or about 30 mg to 70 mg per kg of the weight of a subject.

A composition or treatment of the invention may comprise a unit dosage of at least one scyllo-inositol compound to provide beneficial effects, in particular one or more of the beneficial effects (a) to (t) set out herein. A "unit dosage" or "dosage unit" refers to a unitary i.e., a single dose which is capable of being administered to a patient, and which may be readily handled and packed, remaining as a physically and chemically stable unit dose comprising either the active agents as such or a mixture with one or more solid or liquid pharmaceutical excipients, carriers, or vehicles.

A subject may be treated with a scyllo-inositol compound or composition or formulation thereof on substantially any desired schedule. A composition of the invention may be administered one or more times per day, in particular 1 or 2 times per day, once per week, once a month or continuously. However, a subject may be treated less frequently, such as every other day or once a week, or more frequently.

A scyllo-inositol compound, composition or formulation of the invention may be administered to a subject for about or at least about 1 week, 2 weeks to 4 weeks, 2 weeks to 6 weeks, 2 weeks to 8 weeks, 2 weeks to 10 weeks, 2 weeks to 12 weeks, 2 weeks to 14 weeks, 2 weeks to 16 weeks, 2 weeks to 6 months, 2 weeks to 12 months, 2 weeks to 18 months, or 2 weeks to 24 months, periodically or continuously.

In an aspect, the invention provides a regimen for supplementing a human's diet, comprising administering to the human a supplement comprising a scyllo-inositol compound, or nutraceutically acceptable derivatives thereof. A subject may be treated with a supplement at least about every day, or less frequently, such as every other day or once a week. A supplement of the invention may be taken daily but consumption at lower frequency, such as several times per week or even isolated doses, may be beneficial.

In a particular aspect, the invention provides a regimen for supplementing a human's diet, comprising administering to the human about 25 to about 200 milligrams of a compound of the formula Ia or Ib, or nutraceutically acceptable derivatives thereof on a daily basis. In another aspect, about 50-100 milligrams of a compound of the formula Ia or Ib is administered to the human on a daily basis.

A supplement of the present invention may be ingested with or after a meal. Thus, a supplement may be taken at the time of a person's morning meal, and/or at the time of a person's noontime meal. A portion may be administered shortly before, during, or shortly after the meal. For daily consumption, a portion of the supplement may be consumed shortly before, during, or shortly after the human's morning meal, and a second portion of the supplement may be consumed shortly before, during, or shortly after the human's noontime meal. The morning portion and the noontime portion can each provide approximately the same quantity of a scyllo-inositol compound. A supplement and regimens described herein may be most effective when combined with a balanced diet according to generally accepted nutritional guidelines, and a program of modest to moderate exercise several times a week.

In an embodiment, a regimen for supplementing a human's diet is provided comprising administering to the human a supplement comprising, per gram of supplement: about 5 milligram to about 30 milligrams of one or more scyllo-inositol compound or a nutraceutically acceptable derivative thereof. In an embodiment, a portion of the supplement is administered at the time of the human's morning meal, and a second portion of the supplement is administered at the time of the human's noontime meal.

The invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results.

### Example 1

The following methods were used in the studies described in the example:
Mice. Experimental groups of TgCRND8 mice [17, 18] on a C3H/B6 outbred background were initially treated with either epi- or scyllo-cyclohexanehexol 30 mg/day. This initial dosage was chosen based upon the dosage of myo-cyclohexanehexol (6-18 grams/day/adult or 86-257mg/Kg/day) that is typically administered to human patients for various psychiatric disorders [36]. In these dosages, myo-cyclohexanehexol had no toxicity in humans or animals. The studies described herein were repeated using doses of 5mg/Kg/day-100mg/Kg/day, and these alternate doses have generated the same results (data not shown). A cohort of animals (n=10 mice per treatment arm) entered the study at five months of age, and outcomes were then analyzed after one month of treatment. The body weight, coat characteristics and in cage behaviour were monitored. Mannitol was used as a negative control for potential alterations in caloric intake. All experiments were performed according to the Canadian Council on Animal Care guidelines.
Behavioural tests: Morris Water Maze testing was performed as previously described [18]. After non-spatial pre-training, mice underwent place discrimination training for 5 days with 4-trials per day, followed by a cued visible platform to rule out general motivational, learning deficits and motor problems, and a probe trial to evaluate memory. Data were subjected to a mixed model of repeated measures analysis of variance (ANOVA) with treatment (untreated, epi- or scyllo-cyclohexanehexol) and genotype (TgCRND8 versus non-Tg) as 'between-subject' factors. Open field test for motor activity was preformed as described previously [41]. Duration of walking, pausing and grooming were analyzed as indices of spontaneous locomotor activity. Sensorimotor function was examined with an Economex™ accelerating rotarod (Columbus Instruments, Columbus, OH), as described elsewhere [42]. The rod was set to accelerate at a rate of 0.2 r.p.m./s, from an initial, constant speed of 5 r.p.m.. Latency to fall was recorded in four daily trials, conducted at 30 min intervals.
All mice were trained for seven days before testing. The test day performance score for each animal was obtained by summing its latency to fall over the four trials
**Cerebral amyloid burden**. Brains were removed and one hemisphere was fixed in 4% paraformaldehyde and embedded in paraffin wax in the mid sagittal plane. To generate sets of systematic uniform random sections, 5 µm serial sections were collected across the entire hemisphere. Sets of sections at 50 µm intervals were used for analyses (10-14 sections/set). Plaques were identified after antigen retrieval with formic acid, and incubation with primary anti-Aβ antibody (Dako M-0872), followed by secondary antibody (Dako StreptABCcomplex/horseradish kit). End products were visualized with DAB and were counter-stained with luxol fast blue. Amyloid plaque burden was assessed with Leco IA-3001 image analysis software interfaced with Leica microscope and Hitachi KP-M1U CCD video camera. Openlab imaging software (Improvision, Lexington, MA) was then used to convert micrographs to binary images for plaque number and plaque area determinations. Vascular amyloid burden was defined as amyloid originating from or surrounding blood vessels and was analysed similarly.
**Plasma and Cerebral Aβ Content.** Hemi-brain samples were homogenized in a buffered sucrose solution, followed by either 0.4% diethylamine/100mM NaCl for soluble Aβ levels or cold formic acid for the isolation of total Aβ. After neutralization, the samples were diluted and analyzed for Aβ40 and Aβ42 using commercially available kits (BIOSOURCE International). Each hemisphere was analyzed in triplicate and the mean values ± SEM reported. Western blot analyses were performed on all fractions using urea gels for Aβ species analyses [43]. Aβ was detected using 6E10 (BIOSOURCE International) and Enhanced Chemiluminenscence (Amersham).
**Gliosis Quantitation**. Five randomly selected, evenly spaced, sagittal sections were collected from paraformaldehyde-fixed and frozen hemispheres of treated and control mice. Sections were immunolabelled for astrocytes with anti-rat GFAP IgG₂ₐ (Dako; diluted 1:50) and for microglia with anti-rat CD68 IgG_{2b} (Dako;' 1:50). Digital images were captured using a Coolsnap digital camera (Photometrics, Tuscon, Arizona) mounted to a Zeiss, Axioscope 2 Plus microscope. Images were analysed using Openlab 3.08 imaging software (Improvision, Lexington MA).
**Survival Census:** The probability of survival was assessed by the Kaplan-Meier technique [44], computing the probability of survival at every occurrence of death, thus making it suitable for small sample sizes. For the analyses of survival, 35 mice were used for each treatment group. The Tarone-Ware test was used to assess effects of treatments.
**Analysis of APP in brain**. Mouse hemi-brain samples were homogenized and spun at 109,000 x g, in 20mM Tris pH7.4, 0.25M sucrose, ImM EDTA and ImM EGTA, and a protease inhibitor cocktail, mixed with 0.4%DEA (diethylamine)/100mM NaCl. The supernatants were analysed for APPs levels by Western blotting using mAb 22C11, while the pellets were analysed for APP holoprotein with mAb C1/6.1 as previously described [17,18].
**Soluble Aβ oligomer Analyses**. The levels of soluble Aβ oligomers were measured by a dot blot assay with anti-oligomer specific antibodies [24]. Briefly, oligomers were solubilised from one hemi-brain in PBS in the presence of protease inhibitor cocktail (Sigma). After centrifugation at 78,500 x g for 1 hr at 4°C, the supernatants were analysed. Protein content was determined by the BCA protein assay (Pierce). Two µg of total protein was spotted onto nitrocellulose, blocked with 10% non-fat milk in TBS before incubation with the biotinylated oligomeric specific antibody. Blots were incubated with streptavidin-HRP and ECL chemiluminescence kit. Soluble and fibrillar Aβ42 were used as negative controls and synthetic oligomeric
Aβ42 was used as a positive control [23]. Control samples were re-identified after oligomeric antibody was stripped and re-probing with the anti-Aβ antibody 6E10.
**Long Term Potentiation**. Field potentials were recorded in CA1 of mouse hippocampus by standard procedures [45,46]. Swiss Webster mice between the ages of P16 and P26 were anesthetized with isoflurane. The brain was rapidly removed and placed in ice cold oxygenated sucrose-CSF containing (in mM): 248 sucrose, 2 KCI, 2 MgSO₄, 1.24 NaH₂PO₄, 1 CaCl₂, 1 MgCl₂, 26 NaHCO₃, 10 D-glucose, pH 7.4, ∼315 mOsmol [47]. The hippocampus from each hemisphere was isolated and 350µm coronal sections were made. The slices were transferred to a holding chamber containing NaCl-CSF (in mM: 124 NaCl, 2 KCI, 2 MgSO₄, 1.25 NaH₂PO₄, 2 CaCl₂, 26 NaHCO₃, 10 D-glucose, pH 7.4, ∼ 310 mOsmol) and allowed to recover for more than 1 hour. Once placed in the chamber, slices were continuously perfused by a closed loop containing 15 ml of ACSF to conserve the oligomeric Aβ. After 20 minutes of stable baseline, 1 ml of 15x concentrated 7PA2 conditioned medium ± 1.25 µM scyllo-cyclohexanehexol was added to the perfusion loop. A bipolar stimulating electrode (World Precision Inst.) was placed in the Schaffer collaterals to deliver baseline stimuli and tetani. A borosilicate glass recording electrode (2-4 MΩ) containing ACSF was positioned approximately 75-200 µm from the stimulating electrode. The intensity of the stimulus (typically between 10-20 µAmps) was set to obtain 25-40% of the maximal field potential response. Test stimuli were delivered at 0.05Hz. To induce LTP, 4 tetani (100 Hz for 1 second) were delivered 5 minutes apart. Field potential responses were amplified 10x using an Axopatch 200B.
The data was sampled at 10 kHz and filtered at 2kHz. Traces were analyzed using pClamp 9.2. The slope of the field potential was estimated using approximately 10-60% of the total response.

### Synaptophysin Quantification.

Synaptophysin immunohistochemical staining was performed on 3 evenly spaced saggital sections of paraformaldehyde-fixed treated and control mice. Sections were immunolabelled for synaptophysin with anti-synaptophysin IgG (1:40; Roche, Laval, PQ). Digital images were captured and analyzed as described above. Within each section, three randomly chosen 100 µm² areas of the CA1 region of the hippocampus were counted for synaptophysin reactive cell bodies and boutons. The results are expressed as the mean of the number of reactive bodies and boutons per 100 µm² [48, 49].

### Results

To assess their effectiveness *in vivo,* inositol compounds were administered to a robust murine model of Alzheimer's disease (TgCRND8) [17,18]. TgCRND8 mice express a human amyloid precursor protein transgene (APP₆₉₅) bearing two missense mutations that cause AD in humans (KM670/671NL and V717F). At about three months of age, the mice display progressive spatial learning deficits that are accompanied both by rising cerebral Aβ levels and by increasing numbers of cerebral extracellular amyloid plaques [17]. By six months of age, the levels of Aβ and the morphology, density and distribution of the amyloid plaques in the brain ofTgCRND8 mice are similar to those seen in the brains of humans with well-established AD [17]. As in human patients with AD, the biochemical, behavioural and neuropathological features of the mouse model are accompanied by accelerated mortality [17, 18].

The TgCRND8 mice and non-transgenic littermates were assigned to sex- and age-matched cohorts that were then used to test the effectiveness of the cyclohexanehexol stereoisomers as a therapeutic (with treatment delayed until five months of age and continued for one month until six months of age). The mice were randomly assigned to receive active compound (1,2,3,4,5/6- (epi-) cyclohexanehexol or 1,3,5/2,4,6- (scyllo-) cyclohexanehexol administered orally), mock therapy (mannitol), or no therapy. The endpoints were cognitive function, brain Aβ levels, and neuropathology. 1,2,3,5/4,6-(myo-) cyclohexanehexol was not included in these studies because prior *in vitro* studies [16] had indicated that myo-cyclohexanehexol was only weakly effective, and because pilot *in vivo* studies showed no significant benefit (data not shown). Over the course of these experiments, observers were unaware of genotype or treatment group.

### Cyclohexanehexol Stereoisomers Reverse Established Cerebral Amyloid Deposition

Most AD patients will seek treatment only after they have become symptomatic, i.e., at a time when Aβ oligomerization, deposition, toxicity and plaque formation are already well advanced. To assess whether cyclohexanehexol stereoisomers could abrogate a well-established AD-like phenotype, the start of treatment of the TgCRND8 mice was delayed until five months of age. At this age, TgCRND8 mice have significant behavioural deficits, accompanied by profuse Aβ peptide and plaque burdens [17]. Cohorts of TgCRND8 and non-Tg littermates (10 mice per cohort) were either treated for 28 days with epi-cyclohexanehexol or with scyllo-cyclohexanehexol, or were left untreated. The dosage and oral administration of compounds, and the neurochemical and neuropathological assays used for these experiments were the same as those employed in the initial prophylactic experiments. Mortality curves were not generated for this cohort of animals because the brevity of the trial resulted in too few deaths in the untreated TgCRND8 mice to generate meaningful data.

Spatial learning in these mice was compared between six month old TgCRND8 mice that had been treated with epi-cyclohexanehexol or with scyllo-cyclohexanehexol or that were untreated for 28 days. The performance of six month old TgCRND8 mice that had been treated with epi-cyclohexanehexol for 28 days was not significantly different from that of untreated TgCRND8 littermates (F_{1,15}=3.02; p=0.27; Figure 1A), and was significantly poorer than the performance of their non-Tg littermates (F_{1,14}=11.7, p=0.004; Figure 1C). Furthermore, the probe trial confirmed that epi-cyclohexanehexol treated TgCRND8 mice were not statistically different from untreated TgCRND8 mice (p=0.52; Figure 1E). Epi-cyclohexanehexol had no significant impact on brain Aβ40 or Aβ42 levels, percent area of the brain covered with plaques, or plaque number in animals with pre-existing disease (Table 1).

The 28-day treatment of five month old TgCRND8 mice with scyllo-cyclohexanehexol resulted in significantly better behavioural performance compared to untreated TgCRND8 mice (p=0.01). Indeed, the cognitive performance of these scyllo-cyclohexanehexol-treated TgCRND8 mice was indistinguishable from that of their non-Tg littermates (F_{1,13}=2.9, p=0.11; Figure 1B, D). This beneficial effect of cyclohexanehexol treatment was not due to non-specific effects on behavioural, motor, or perceptual systems because cyclohexanehexol treatment had no effect on the cognitive performance of non-Tg mice (F_{2,19}=0.98; p=0.39). In the probe trial, the annulus-crossing index showed a significant improvement in memory for scyllo-cyclohexanehexol treated TgCRND8 mice that was not statistically different from non-Tg littermates (p=0.64; Figure 1E). In a separate cohort of mice and using % time in target quadrant as an alternate measure, scyllo-cyclohexanehexol treated TgCRND8 mice were not statistically different from non-Tg littermates (p=0.28; data not shown). The beneficial effects of scyllo-cyclohexanehexol were not due to alteration of sensorimotor behaviour. Scyllo-cyclohexanehexol had no effect on grooming or activity of TgCRND8 mice in comparison to both untreated TgCRND8 mice (F_{1,9}=0.25; p=0.63) and non-Tg littermates (F_{1,12}=0.02; p=0.89) in the open field test (supplemental data). Similarly, Rotarod testing revealed no difference between scyllo-cyclohexanehexol treated and untreated TgCRND8 mice (p=0.42) or between treated TgCRND8 and treated or untreated non-Tg littermates (p=0.79) in sensorimotor function. In agreement with the results of the prophylactic study, a 28 day course of scyllo-cyclohexanehexol at 5 months of age also: 1) reduced brain levels of Aβ40 and Aβ42 (e.g. insoluble Aβ40 = 29±2.3% reduction, p<0.05; insoluble Aβ42 = 23 ± 1.4% reduction, p<0.05); and 2) significantly reduced plaque number, plaque size, and percent area of the brain covered in plaques (plaque number =13 ± 0.3% reduction, p<0.05; plaque size =16±0.4% reduction, p = 0.05; percent area of the brain covered by plaques = 14±0.5% reduction, p<0.05; Table 1; Figure 1C-D). These results are comparable in effect to those of the six month prophylactic studies.

In sum the data show that scyllo-cyclohexanehexol, and to a lesser degree, epi-cyclohexanehexol, can prevent and reverse the AD-like phenotype in TgCRND8 mice, reducing cognitive deficits, amyloid plaques, amyloid angiopathy, Aβ-induced inflammatory response, and accelerated mortality. These effects are likely direct effects of the compounds within the CNS because: 1) the compounds are transported across the blood brain barrier by facilitated transport [20, 22]; and 2) their presence can be demonstrated in the brain tissue of treated mice by gas chromatography-mass spectrometry [23] (data not shown).

There was no change in the levels of APP holoprotein, APP glycosylation, APPs-α or APPs-β, or Aβ speciation (i.e. Aβ1-38 levels) in brain homogenates from treated and untreated TgCRND8 mice (data not shown). Similarly, the peripheral distribution of Aβ as measured by plasma Aβ42 levels were not different between treated and untreated TgCRND8 mice. Plasma Aβ42 levels in the cohort of TgCRND8 mice following 28 days of cyclohexanehexol therapy at five months of age were: untreated = 1144 ± 76 pg/ml; epi-cyclohexanehexol = 1079 ± 79 pg/ml; scyllo-cyclohexanehexol = 990 ± 73 pg/ml; p=0.87. The absence of alterations in peripheral/plasma Aβ42 may be relevant because plasma Aβ levels were also unchanged in patients who developed a strong antibody response and an apparent clinical improvement following Aβ immuno-therapy [4].

To directly address the possibility that the cyclohexanehexol stereoisomers inhibit Aβ oligomerization in the brain, an activity that they clearly have *in vitro* [15,16], a dot blot immunoassay [24] was used to measure levels of Aβ oligomers in the brains of treated and untreated TgCRND8 mice. This assay employs an antibody that selectively identifies oligomeric Aβ species [24]. The levels of soluble Aβ oligomers were significantly reduced in the brain of treated mice, and these reductions were commensurate with the degree of behavioural and neuropathological improvements induced by these compounds (Figure 2). Aβ oligomers were not significantly reduced after one-month treatment with epi-cyclohexanehexol in the five month old TgCRND8 mice with existing pathology (56 ± 4 pixels in untreated TgCRND8 versus 47 ± 2 pixels in epi-cyclohexanehexol treated TgCRND8, p=0.12). Delayed 28-day treatment with scyllo-cyclohexanehexol at five months of age also caused a 30% reduction in soluble Aβ oligomers (63 ± 3 pixels in untreated TgCRND8 versus 45 ± 2 in scyllo-cyclohexanehexol treated TgCRND8, p=0.008). The dot blots were negative for cross-reactivity to tau, α-synuclein and tubulin, demonstrating specificity of the antibody for Aβ in the TgCRND8 brain homogenates. These results directly demonstrate that scyllo-cyclohexanehexol, but not epi-cyclohexanehexol, decreases the amount of soluble Aβ oligomers in the brain.

To address the possibility that scyllo-cyclohexanehexol inhibits Aβ oligomer-induced neurotoxicity, its effects were determined on both long term potentiation (LTP) in mouse hippocampal slices and on synaptic density as measured by the level of synaptophysin immunoreactivity in the brains of TgCRND8 mice. Hippocampal LTP is a measure of synaptic plasticity, and has been shown to be disrupted by natural cell-derived oligomeric Aβ species [26]. As previously reported in rat [26, 27], soluble Aβ oligomers secreted into the conditioned media of CHO cells stably transfected with human APPV717F (7PA2 cells) inhibited LTP in wild-type mouse hippocampal slices (Figure 2B). However, when the 7PA2-conditioned medium was pretreated *in vitro* with scyllo-cyclohexanehexol, there was a significant recovery of LTP compared with 7PA2-conditioned media alone (p=0.003; Figure 2B). Scyllo-cyclohexanehexol had no direct effect on LTP as scyllo-cyclohexanehexol treated culture media from plain CHO cells that were not transfected with human APP (Figure 2C) and untreated culture media from these cells were indistinguishable from scyllo-cyclohexanehexol treated 7PA2 culture media, i.e., all three samples allowed LTP. The LTP effects were not a result of altered baseline transmission, since scyllo-cyclohexanehexol did not change synaptic response in the absence of a potentiating tetanus (data not shown). In order to correlate this protection of LTP in slice cultures with *in vivo* effects on synaptic function, the level of synaptophysin immunoreactivity was measured in the CA1 region of the hippocampus in scyllo-cyclohexanehexol-treated and untreated TgCRND8 mice. Synaptophysin immunoreactivity is a measure of synaptic density, which is correlated to synaptic function. The levels of synaptophysin were significantly increased. Thus, scyllo-cyclohexanehexol increased the number of synaptophysin reactive boutons and cell bodies in the CA1 region of the hippocampus by 148% for a prophylactic study group (1610 ± 176/100 µm² in untreated TgCRND8 mice versus 2384 ± 232/100 µm² in scyllo-cyclohexanehexol treated TgCRND8 mice; p=0.03) and by 150% for the delayed treatment study (1750 ± 84/100 µm² in untreated versus 2625±124/100 µm² in scyllo-cyclohexanehexol treated TgCRND8 mice; p<0.001). Together, the results of the LTP and synaptophysin studies suggest that in the brain, scyllo-cyclohexanehexol may restore the inhibition of LTP induced by naturally secreted human Aβ oligomers, and allow maintenance of synaptic function.

Scyllo-inositol was also administered to TgCRND8 mice for 2 months, ending at 7 months of age. Sustained effects both on cognition and pathology were observed in these treated animals.

### Example 2

### Investigation into the effects of AZD-103 on cell-derived Aβ oligomers and impact on hippocampal long-term potentiation

The purpose of this study was to investigate the potential therapeutic effects of AZD-103 to neutralize soluble Aβ oligomers which are thought to play an important role in the etiology of Alzheimer's disease. The effects of a scyllo-inositol compound (i.e., AZD-103, a scyllo-cyclohexanehexol) on the small, soluble Aβ oligomers produced by the "7PA2" cells, a CHO cell-line that stably overexpresses APP751_{V717F}, were examined. These cells produce a series of Aβ oligomers, as detected by Western blot. These Aβ oligomers have been shown to profoundly inhibit long-term potentiation (a method for measuring synaptic efficacy and plasticity in laboratory animals) (LTP) in the hippocampus of rodents. Thus the primary goals of this study were to determine whether AZD-103 affects the pattern of Aβ oligomer detected by Western blot (indicative of either disaggregation or epitope masking), and secondly to examine whether AZD-103 could rescue LTP from the adverse effects of Aβ oligomers.

### Objectives:

1) Test the effects of acute application of 1.25µM AZD-103 to 7PA2 conditioned media (CM) just prior to performing LTP experiments. The purpose of this experiment was to determine whether AZD-103 could rescue LTP from fully assembled Aβ oligomers.
2) Test the effects of acute application of 1.25µM chiro and epi enantiomers of AZD-103. The purpose of this experiment was to determine whether the effect is specific to AZD-103 and not less or inactive compounds.
3) Perform a dose response curve with AZD-103 using the LTP paradigm. The aim of this experiment was to estimate an IC₅₀ for AZD-103 in the context of 7PA2 CM.
4) Establish a time-of-incubation curve using a low concentration of AZD-103 in the LTP experimental paradigm. This experiment was designed to determine whether longer co-incubation periods of AZD-103 and 7PA2 CM improved the rescue of LTP.
5) Test whether application of high levels of AZD-103 to hippocampal slices that have already been exposed to 7PA2 CM, could still rescue the LTP. The purpose of this study was to establish whether AZD-103 could reverse the effects of Aβ oligomers once they penetrated the brain tissue.
6) Perform IP/Westem blot analysis on 7PA2 CM that has been treated with a serial dilution of AZD-103 (post-cond). Perform a similar experiment with the addition of AZD-103 directly to the 7PA2 cells prior to conditioning (pre-cond). This experiment was designed to compare the effectiveness of AZD-103 on oligomer stability versus oligomer production.
7) Test whether relatively low doses of AZD-103 are more effective at rescuing LTP when applied pre-cond as compared to post-cond.

### Methods:

*Electrophysiology*: A detailed description of the electrophysiology methods can be found in the publication Walsh et al. Journal ofNeuroscience 25:2455-242. Briefly, 350µm coronal sections were prepared from p16-p28 Swiss Webster mice brains. Field potential recordings were made in the CA1 region of the hippocampus, while stimulating the Schaeffer collaterals. A 20 minute recording in artificial cerebral spinal fluid (ACSF) was performed to establish a stable baseline. During this interval a 1ml aliquot of 15x concentrated 7PA2 CM was thawed at 37°C, at five minutes, 18.75µM AZD-103 was added to this conditioned media, mixed and returned to 37°C. After 15min the 7PA2 CM / AZD-103 mixture was diluted into 15ml of ACSF for a final concentration of 1x 7PA2 CM and 1.25µM AZD-103. The 15ml was then continuously recirculated over the brain slice for an additional 20 minutes to allow the Aβ to penetrate into the tissue. To induce LTP, four 100Hz tetani were delivered every 5 minutes. The slope of the evoked EPSP was followed for 1 hr post-tetanus. The 1 hr time point was the focus of the analysis, since this is the initial stage of LTP which is greatly impacted by Aβ oligomers.

*Preparation of conditioned media*: CHO- or 7PA2 cells were grown to ∼90% confluency. The cells were washed 1x in serum-free DMEM, then incubated overnight (∼15hrs) in 4ml / 10cm dish in serum-free DMEM, pen/strep, I-glutamine with/without AZD-103. The following day, the conditioned media (CM) was collected, spun at 1000xg and treated with complete protease inhibitors (in mg/ml 1 leupeptin, 1 pepstatin, 0.1 aprotinin, 40 EDTA, and 2mM 1/10 phenantroline) for biochemistry experiments or cell culture compatible protease inhibitors (Sigma P1860 1:1000) for electrophysiology experiments. The CM was stored at -80°C until sufficient volumes were collected to complete a "batch" - typically ∼300ml. These samples were then centrifuged in YM-3 centricon filtration units to concentrate the CM 15x. The resulting concentrate was pooled, aliquoted in 1 ml fraction, and stored at -80°C. There is some variability in the 7PA2 CM that occurs from batch to batch (typical inhibition is 120% - 150% of baseline, relative to 200%-220% for CHO- controls) that can be due to several factors such as small differences in the confluency of the cells and passage numbers. Therefore, for any given set of experiments (i.e. dose response curve, time curve, etc), a single batch is prepared and compared to 7PA2 alone within that batch.

*IPlWestern blots*: 8ml of 7PA2 CM were precleared with 40µl of protein A agarose for 30min. The beads were spun down, and 60µl of the polyclonal anti-Aβ antibody R1282 was added to the supernatant with an additional 40µl of protein A agarose. These samples were nutated at 4°C overnight. The beads were washed with a series of buffers 0.5 STEN (sodium chloride, tris, EDTA, NP-40), SDS STEN, STEN. 2x tricine sample buffer was added to the washed beads, which were then boiled, centrifuged, and the resulting supernatant loaded onto 10-20% tricine gels. The proteins were then transferred to nitrocellulose, and probed with the anti-Aβ antibody 6E10.

### Results:

1.25µM AZD-103 applied directly to the CM (post-cond) 15 minutes prior to the application of the media to brain slices, completely rescues LTP from the effects of Aβ oligomers (Figure 3A, 3B and 3C, Table 2). At 120 minutes (60min post tetanus), the slope of the EPSP was found to be 218% of baseline in CHO-/AZD-103 controls. (This is also typical of CHO- alone). As expected, 7PA2 CM significantly inhibited LTP at 60min post tetanus (150% of baseline). However, a 15 minute co-incubation of 1.25µM AZD-103 with 7PA2 CM was sufficient to completely rescue the LTP. The epi enantiomer of AZD-103 was also found to be effective at restoring LTP (although this turned out not to be statistically significant, probably because of the small n), while the chiro enantiomer was not at the 1.25µM concentration.

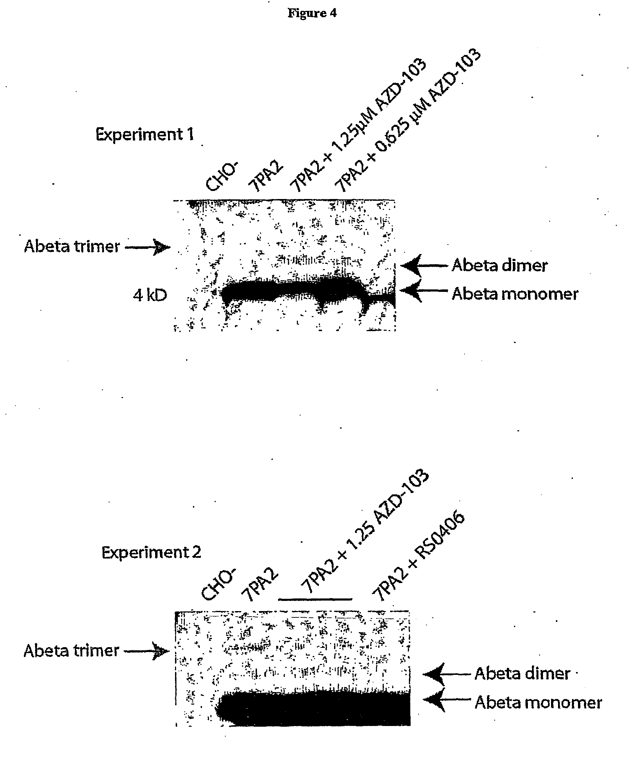

Initial experiments suggested that the application of AZD-103 to CM (post-cond) reduced the detectability of the Aβ trimer on Western blots (Figure 4). Nevertheless, the dimer doublet, seemed unaffected Experiment 1, Figure 4). In the first experiment, two different doses of AZD-103 were tested. The high dose (1.25µM) AZD-103 appeared to reduce the trimer, increase a slightly smaller band, and reduce the monomer, while the dimer was unaffected (Experiment 2, Figure 4). In the second experiment, 1.25µM AZD-103 also reduced the trimer, while leaving the dimer and monomer unaffected. The RS0406 compound was used as a positive control, since it was previously shown that it reduces the production of oligomers when added pre-cond.

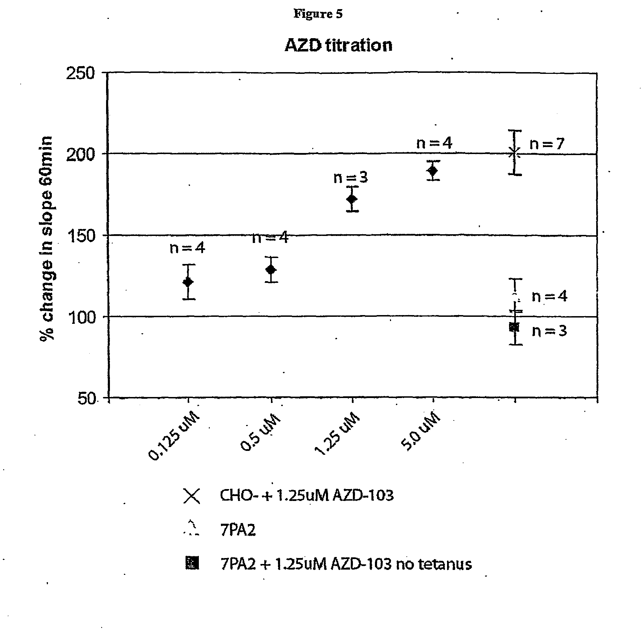

A dose response curve of AZD-103 was performed to establish a range of concentrations of AZD-103 that are effective at rescuing LTP (Figure 5, Table 3). Four different concentrations of AZD-103 (0.125, 0.5, 1.25, and 5.0 µM) were added to 7PA2 CM post-cond. Note that this batch of 7PA2 was slightly more effective at inhibiting LTP (113% baseline for 7PA2 alone) and that 1.25uM AZD-103 was not as effective as in the previous study. Nevertheless, a clear dose response was shown with an IC₅₀ of ∼1µM (with the caveat that this may vary slightly between batches of 7PA2 CM).

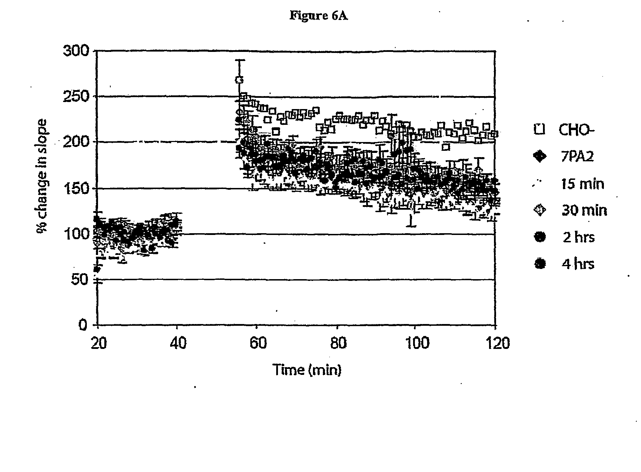

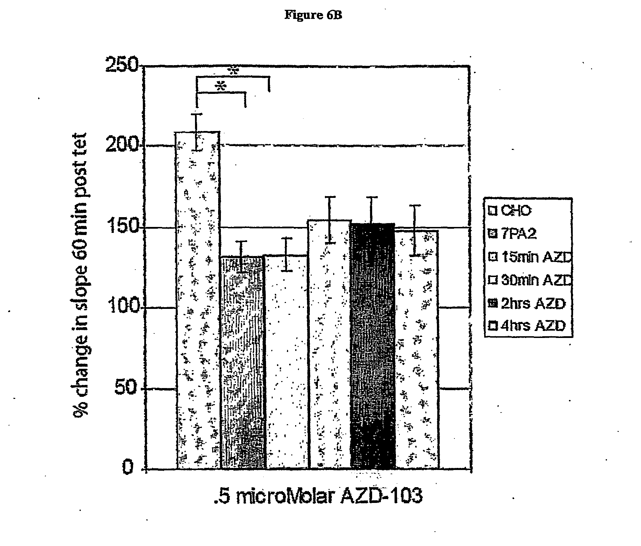

Prolonging the duration of AZD-103 / 7PA2 CM co-incubation did not alter the effect on LTP (Figure 6A and 6B, and Table 4). A relatively low dose of AZD-103 (0.5µM) was selected to determine if longer incubations (15,30,120,240 min) would show improved rescue of LTP. None of these longer incubations was found to be significantly different from 15min or 7PA2 alone. However, all time points except 15 min lost significance as compared to CHO-. This is consistent with a partial effect of 0.5µM on alleviation of LTP inhibition, as observed in Figure 5.

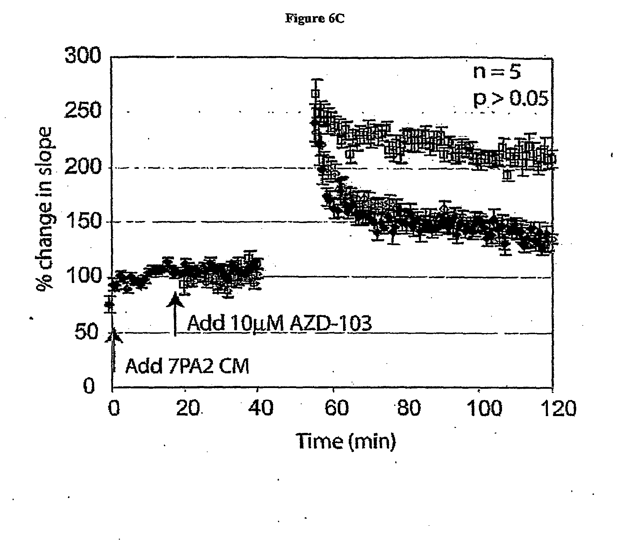

AZD-103 was not effective at reversing the inhibition of LTP by Aβ oligomers once the slice had been perfused with intact oligomers (Figure 6C). A relatively high concentration of AZD-103 (10 µM) was applied to mouse brain slices 20min after perfusion with 7PA2 CM. The slice was then perfused for an additional 10min with AZD-103 / 7PA2 CM. The LTP at 60min post-tetanus was not significantly different from 7PA2 controls. Nevertheless, a relatively low concentration of AZD-103 (0.5µM) applied directly to the 7PA2 cells (pre-cond) did successfully rescue LTP when the CM from these cells was applied to mouse brain slices. Thus, AZD-103 proved more effective at rescuing LTP when applied pre-cond than post-cond.

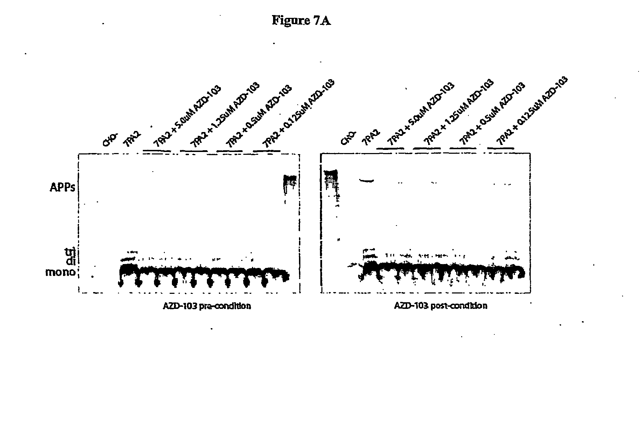

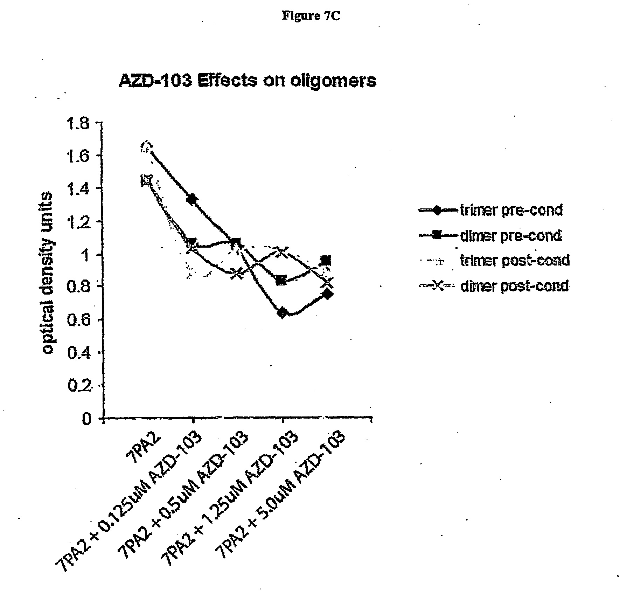

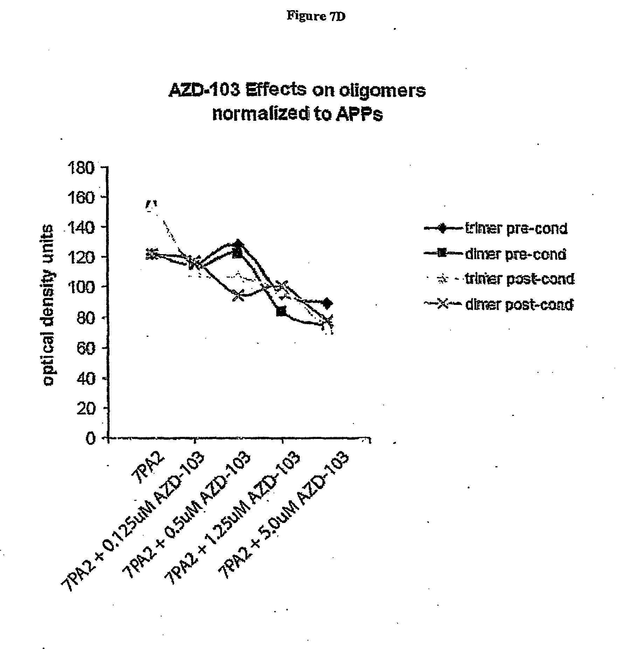

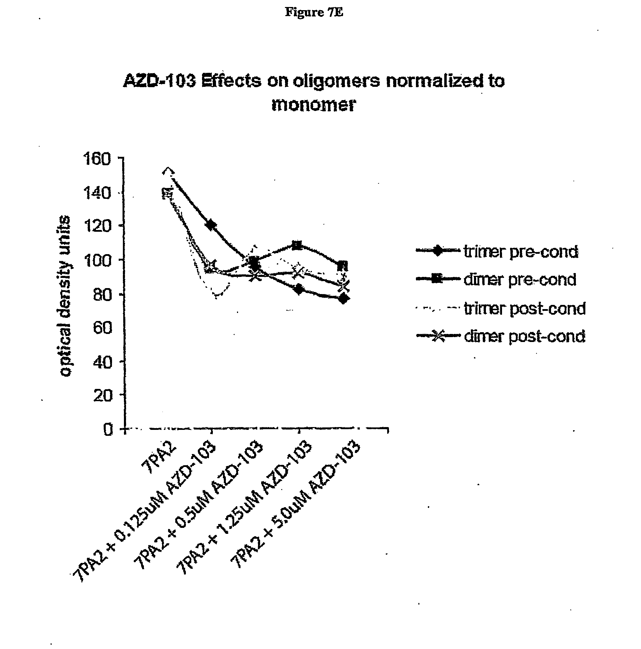

A dose response curve was performed to establish at which concentrations AZD-103 effectively reduced Aβ oligomers as assayed by Western blot (Figures 7A through 7E). For these experiments, AZD-103 was added to 7PA2 cells directly prior to conditioning (pre-cond) as well as to the 7PA2 CM (post-cond). Aβ oligomers are effectively reduced by both pre-cond and post-cond. One challenge in performing these experiments is reducing the variability between samples during the IP washes. Therefore an absolute measure of Aβ trimers and dimers (Figure 7C) was included, as well as measures normalized to APP or Aβ monomer (Figures 7D and 7E). All concentrations of AZD 103 appeared to reduce dimer and trimer levels when measured absolutely and when normalized to monomer. When normalized to APP, the effects of AZD103 may suggest a dose response.

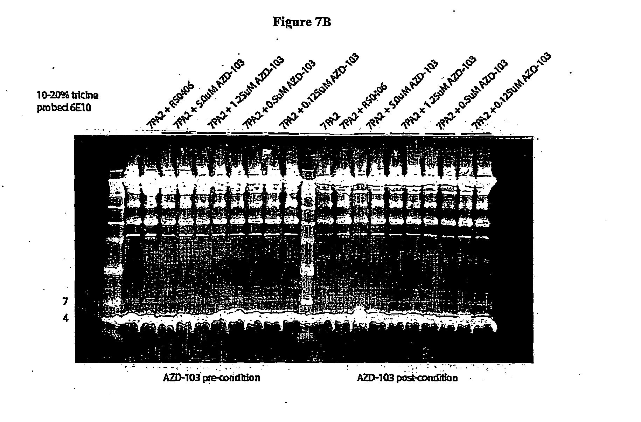

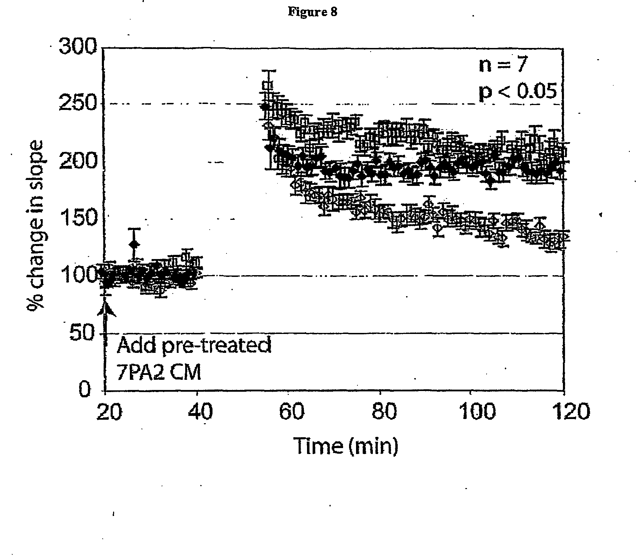

In Figure 7, no difference could be observed in the effect of AZD 103 on oligomers when added directly to 7PA2 cells (pre-conditioning, compared to incubation of AZD103 with 7PA2 CM (post-conditioning). Nevertheless, the effects of 7PA2 CM in the LTP paradigm when cells had been pretreated (pre-cond) with AZD-103 were tested. A relatively low concentration was used (0.5 µM) - a level which had shown only partial efficacy with the post-conditioning regimen. 0.5 µM AZD103 applied directly to cells prior to conditioning had a profound impact on the ability of the CM to inhibit LTP. The % change in slope at 120 minutes was similar to that observed in the absence of oligomers (CHO-) (Figure 8; compare with Figure 6 0.5 µM with a post-conditioning regiment). These results suggest that AZD-103 had additional benefits at lower concentrations when applied directly to the oligomer-producing cells rather than to the post-conditioned media.

### Conclusions:

The main conclusion from the study is that AZD-103 proved highly effective at neutralizing the short term effects of Aβ oligomers on synaptic function in the hippocampus of mice (Figure 3).

The standard assay of long-term potentiation (LTP) has been extensively described in the literature, and is widely accepted as a measure of synaptic efficacy and plasticity in the brain. The cellular and molecular basis of LTP is thought to employ the same mechanisms that are necessary for learning and memory in humans. Thus the ability of Aβ oligomers to interfere with LTP, is likely to mimic similar processes that impair memory in Alzheimer's patients. Based on this assumption, the main effect of AZD-103 to restore LTP in the mouse hippocampus, is consistent with the supposition that AZD-103 may have similar functions and provide similar benefits to Alzheimer's patients.

A second interest of this study was to gain insight into how AZD-103 modifies Aβ oligomers to render them impotent against LTP. Initial experiments (some of which are included in Figure 4) suggested that AZD-103 may mask or disassemble Aβ trimers, which are particularly potent in inhibiting LTP. The titration experiments shown in Figure 7 generally support this conclusion.

AZD-103 proved to be more effective at rescuing LTP (Figure 5, 8) when applied to 7PA2 cells (pre-cond) rather than to 7PA2 CM (post-cond). While AZD103 can reduce dimers and trimers directly, it has an enhanced/further effect when incubated with the cells which secrete the oligomers.

AZD-103 did not restore LTP when added after perfusing the brain slices with 7PA2 CM (Figure 6). However, even if AZD-103 is unable to reverse pre-existing damage to the brain, it may effectively neutralize newly generated Aβ oligomers, and prevent additional damage. This may allow other restorative mechanisms to operate more effectively and improve the prognosis over a longer time period than can be measured by the assay.

For this study, WT mice and an added exogenous source of cell-derived Aβ oligomers were used. One advantage of this experimental methodology is that the acute effects of Aβ on synaptic function can be studied, and the compensatory effects that may resist Aβ toxicity and secondary and tertiary consequences of the initial Aβ insult are reduced. A second advantage is that the cell-derived CM contains a rich assortment of highly stable Aβ oligomers, which are difficult to reproduce with synthetic Aβ. Finally, the levels of Aβ necessary to impair LTP are extremely low (initial estimates are in the high picomolar range) which much more closely approximates the levels of Aβ seen in Alzheimer's patients. Therefore, this is a discerning system for testing AZD-103's ability to interfere with the detrimental effects of Aβ oligomers on synaptic function.

No direct evidence of AZD-103 toxicity was observed (7PA2 cells appeared healthy in the presence of AZD-103); APPs expression was normal even when Aβ trimers were reduced; and, no adverse effects on LTP in CHO- / AZD-103 conditions were observed (see Figure 3)).

### Summary

Natural, cell-derived oligomers of human amyloid β-protein (Aβ) profoundly inhibit long-term potentiation (LTP) in the hippocampus of rodents *in vivo.* These oligomers also impair the recall of a learned behavior in rats, a finding that supports the hypothesis that soluble, low-n oligomers of Aβ impair memory and could contribute to early symptoms of Alzheimer's disease.

The principal goal in this study was to determine whether the cyclohexanehexol, AZD-103 could offer therapeutic benefit by neutralizing the inhibitory effects of human Aβ oligomers on synaptic function. Wild-type mouse hippocampal slices were perfused with conditioned medium (CM) containing secreted Aβ oligomers (at low nM concentrations) that had been treated with AZD-103. At∼1-2 µM concentrations, AZD-103 completely rescued LTP, whereas an inactive enantiomer conferred no benefit. Even lower concentrations of AZD-103 were effective when applied directly to the Aβ-secreting cells. Analysis of Aβ oligomers in the CM by IP/Western suggested that AZD-103 reduces their levels.

AZD-103 rescues hippocampal LTP from the inhibitory effects of soluble Aβ by reducing preformed oligomers. Thus, AZD-103 may interfere with an early pathogenic step in AD.

### Example 3

### Effects of AZD103 on amyloid-β oligomer-induced cognitive deficits

### Purpose:

Various compounds, including AZD 103, were tested in an Alternating Lever Cyclic Ratio rat model of Alzheimer's disease (AD). This highly sensitive model has been able to detect cognitive deficits due to direct injection of amyloid (Aβ) oligomers into rat brain. Small molecule compounds are administered concurrent with Aβ oligomers known to adversely affect cognition and their ability to counteract the oligomer-induced cognitive decline are assessed.

The Aβ oligomers are naturally produced by cells transfected with genes that over-produce the amyloid precursor protein (APP). APP is cleaved by secretase(s) and the cells construct the oligomeric Aβ into molecules ranging from 2 to 12 amyloid proteins (2 mer to 12 mer) and secrete it into the culture medium (CM) of cultured cells [5]. In addition, oligomeric Aβ is extracted from brain homogenate taken from transgenic mice (Tg2576) transfected with the Swedish APP mutation [51]. Aβ oligomers are soluble, do not form fibrils/plaques, and are stable in solution. Whether from CM or brain homogenate, the oligomers are purified by size exclusion chromatography (SEC) into specific molecular weight categories that have been shown to adversely affect cognition [50]. These purified oligomeric forms of Aβ, at physiological concentrations relevant to those found in AD, are injected into the rat's lateral ventricle through an indwelling cannula while the animals are awake and moving. Two hours after injection the rats are tested under sensitive cognitive assay.

The assay, the Alternating Lever Cyclic Ratio (ALCR) test, has proven to be much more sensitive than previously published methods for measuring drug effects on cognitive function [52, 53]. In this task, rats must learn a complex sequence of lever-pressing requirements in order to earn food reinforcement in a two-lever experimental chamber. Subjects must alternate between two levers by switching to the other lever after pressing the first lever enough to get food reward. The exact number of presses required for each food reward changes, first increasing from 2 responses per food pellet up to 56 presses per food pellet, then decreasing back to 2 responses per pellet. Intermediate values are based on the quadratic function, *x*² - *x*. One cycle is an entire ascending and descending sequence of these lever press requirements (e.g., 2, 6, 12, 20, 30, 42, 56, 56, 42, 30, 20, 12, 6, and 2 presses per food reward). Six such full cycles are presented during each daily session. Errors are scored when the subject perseveres on a lever after pressing enough to get the food reward, i.e., does not alternate (a Perseveration Error), or when a subject switches levers before completing the response requirement on that lever (a Switching Error).

### Methods:

Oligomeric Aβ: Prepared from transfected Chinese Hamster Ovary Cells (7PA2 cells). These cells secrete oligomeric Aβ into the culture medium (CM) at physiological levels. Aβ oligomers was also derived from Tg2576 mouse brain and purified by size exclusion chromatography. Samples of oligomeric Aβ were characterized by Western Blot Analysis. Appropriate control compounds were produced and tested for each active Aβ oligomeric configuration.
Rats: Forty (40) rats were trained under ALCR for approximately 3 months until their error rates are stable. Training sessions were conducted 5 days each week.
Surgery: After training, all rats received a single 28 ga. cannula that was permanently affixed to the skull, and aimed at the lateral ventricle (divided equally between left and right). Rats were allowed 5 days to recover from surgery.
Administration: *In vivo* administration of AZD103 to rats was performed by dissolving AZD103 into drinking water. Different concentrations were prepared, and the average daily water intake used to target specific average daily dose levels. These dose levels were therefore approximate.
Testing: AZD 103 was tested against Aβ oligomers known to disrupt cognitive function. Two general procedures were incorporated.
   1. AZD 103 was incubated with the injectate medium containing Aβ oligomers prior to assessing it's affect on ALCR Appropriate control injections were included un-incubated (untreated) Aβ oligomers as well as AZD 103 were injected ICV.
   2. Rats were treated with AZD 103, administered in drinking water, for at least 3-4 days prior to testing ICV injection of Aβ oligomers preparations known to affect cognitive function under ALCR

Treatments were assessed in the following order:
1. ICV 7PA2 CM alone
2. ICV AZD 103 alone
3. ICV *ex vivo* incubated 7PA2 CM with AZD 103
   All rats received treatments 1-3 in randomized order.
4. ICV 7PA2 CM after 4 days treatment with PO AZD103 30 mg/kg/day.
5. ICV 7PA2 CM after 4 days treatment with PO AZD103 100 mg/kg/day.
6. ICV 7PA2 CM after 4 days treatment with PO AZD103 300 mg/kg/day.
7. ICV 7PA2 CM after 4 days with no treatment.

Error Rate Analysis: All error rates under AZD 103 were compared to baseline error rates consisting of at least 3-4 non-treatment days prior to injections. This is a repeated measure within subject design that produces maximum power to detect changes in the error rates.
Histology: Upon completion of the study, 20 rat brains were banked. The other 20 brains were evaluated histologically for inflammation, gliosis and cannula placement. Perfusion-fixed brains from these 20 animals were drop fixed in formalin and right and left hemispheres were processed separately. Serial hematoxylin and eosin stained sections were used to evaluate for cannula placement. These same hemibrains were evaluated for inflammation (neutrophils/lymphocytes/macrophages), gliosis (microglial and astrocytic) and neuron loss using standard hematoxylin and eosin staining as well as specific markers for gliosis as needed.

The opposite hemisphere was preserved in formalin for confocal immunohistochemical fluorescent photomicrographs (GFAP, Neu-N, DAPI, propidium iodide) should inflammatory changes be considered significant upon H & E analysis.

### Results

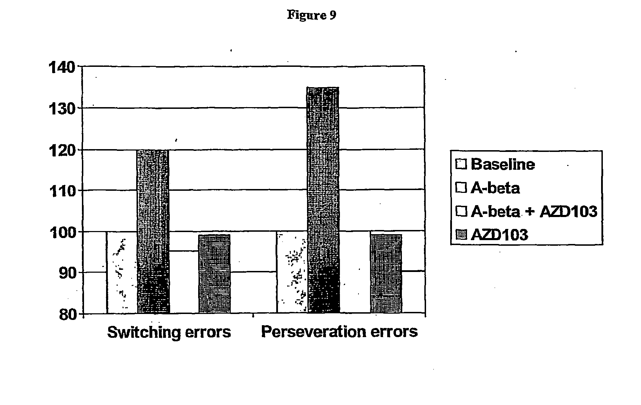

The results are presented in Table 5. Both types of errors were increased by the infusion of Aβ oligomers (120% and 135% of baseline errors, for switching and perseveration errors (p=0.011 and 0.007) respectively). When the oligomers were incubated with AZD103 prior to infusion, the number of errors returned to baseline (95% and 100% of baseline errors, for switching and perseveration, (p=0.50 and 0.99) respectively). AZD103 is therefore able to prevent the Aβ oligomers from causing cognitive dysfunction. AZD103 administered without oligomers had no impact on performance, demonstrating that the drug was not providing non-specific cognitive enhancement (Figure 9).

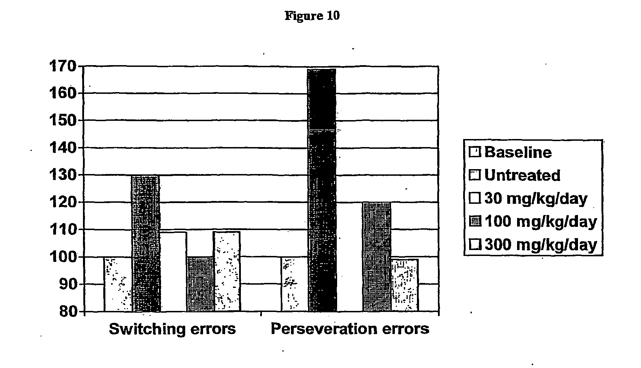

The ability of orally-dosed AZD103 to prevent amyloid-induced acute cognitive dysfunction was then investigated. Rats were administered AZD103 (30, 100, and 300 mg/kg/day in drinking water) for four days at each dose level. Aβ was then infused through the cannula into the brain. After two hours, the rats underwent the ALCR test. Aβ oligomers caused significant increases in errors when animals were untreated (switching errors: 130%of baseline errors, p=0.003; perseveration errors: 169% of baseline errors, p=0.009). However, the error rate was restored to baseline levels by all dose levels of AZD103 (for the 30, 100 and 300 mg/kg/day doses, the % of baseline errors was 109%, 100%, 109% for switching errors; and 120%, 120%, 99% for perseveration errors, respectively) (Figure 10).

AZD103 is therefore effective at alleviating cognitive dysfunction that is caused by acute exposure to amyloid in the brain of rats. The ex vivo incubation demonstrates that AZD103 is capable of neutralizing the deleterious cognitive effects of Aβ in rats. Thus, the *in vivo* administration data show that AZD103 is sufficiently brain penetrant following oral dosing in rats to express its therapeutic potential. The study demonstrates the potential of AZD103 to treat amyloid-induced cognitive disorders.

The present invention is not to be limited in scope by the specific embodiments described herein, since such embodiments are intended as but single illustrations of one aspect of the invention and any functionally equivalent embodiments are within the scope of this invention. Indeed, various modifications of the invention in addition to those shown and described herein will become apparent to those skilled in the art from the foregoing description and accompanying drawings. Such modifications are intended to fall within the scope of the appended claims.

All publications, patents and patent applications referred to herein are incorporated by reference in their entirety to the same extent as if each individual publication, patent or patent application was specifically and individually indicated to be incorporated by reference in its entirety. The citation of any reference herein is not an admission that such reference is available as prior art to the instant invention.

**Table 1**

| Cyclohexanehexol treatment for 28 days decreases brain Aβ40 and Aβ42 Levels and amyloid plaques at 6 months of age. | | | | | | | |
|---|---|---|---|---|---|---|---|
| | Aβ40 (ng/gm wet brain ± sem) | | Aβ42 (ng/gm wet brain ± sem) | | Total Plaque | Total Plaque | Mean Plaque |
| | Soluble | Insoluble | Soluble | Insoluble | Count | Area (µm²) | Size (µm²) |
| Control | 204±4 | 4965±457 | 426±14 | 14503±1071 | 1441±29 | 486002±16156 | 401±14 |
| Epi-cyclohexanchexol | 264±11 | 3637±113* | 540±14 | 12830±330 | 1342±114 | 459706±49966 | 346±6 |
| Scyllo-cyclohexanehexol | 178±11 | 3527±241* | 374±23 | 11115±647* | 1260±27* | 420027±14986* | 336±6* |

| | | | | | | | |
|---|---|---|---|---|---|---|---|
| ANOVA with Fisher's PLSD, * p<0.05. | | | | | | | |

**Table 2**

| % change in EPSP slope 60 minutes after tetanus, when the slices had been perfused with pre-incubated mixture of the indicated CM and test article; as illustrated in Figure 3C. | | | | |
|---|---|---|---|---|
| **Conditioned Media** | **AZD conc** | **Avg** | **SEM** | **n** |
| CHO- | 1.25µM | 217.785 | 16.0585 | 8 |
| 7PA2 | | 150.325 | 9.55269 | 10 |
| 7PA2 | 1.25µM | 217.76 | 14.6745 | 8 |
| | | | | |
| | **Epi conc** | | | |
| 7PA2 | 1.25µM | 202.3289 | 15.15965 | 4 |
| | **Chiro** | | | |
| 7PA2 | 1.25µM | 125.6881 | 9.247232 | 6 |
| CHO- | 1.25µM | 186.1729 | 14.70473 | 4 |

| | | | | |
|---|---|---|---|---|
| "Avg" is the % change in EPSP slope. | | | | |

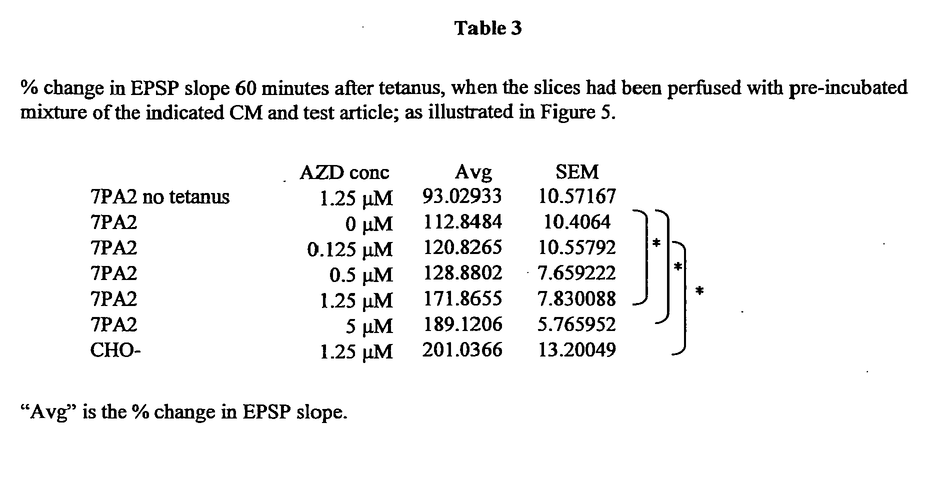

**Table 4**

| % change in EPSP slope 60 minutes after tetanus, when the slices had been perfused with pre-incubated mixture of the indicated CM for the specified time (CHO CM was incubated with AZD103 for 30 minutes) and test article; as illustrated in Figure 6B. "Avg" is the % change in EPSP slope. | | | | |
|---|---|---|---|---|
| | AZD conc | AVG | SEM | n |
| CHO | 0.5 µM | 208.3086 | 11.21214 | 6 |
| 7PA2 | 0.5 µM | 131.4844 | 9.260153 | 6 |
| 15min | 0.5 µM | 132.824 | 10.24573 | 7 |
| 30min | 0.5 µM | 154.605 | 14.18698 | 9 |
| 2hrs | 0.5 µM | 151.8599 | 16.98528 | 7 |
| 4hrs | 0.5 µM | 147.7836 | 15.60832 | 4 |

**Table 5**

| Error rate following icv infusion of Aβ oligomers, when either preincubated ex vivo with AZD103, or when AZD103 is administered p.o. | | | | |
|---|---|---|---|---|
| | Switching | | Perseveration | |
| | % errors | P= | % errors | P= |
| **Ex vivo incubation** | | | | |
| 1 Baseline | 100 | | 100 | |
| 2 Oligomers alone | 120 | 0.011 | 135 | 0.007 |
| 3 AZD103 alone | 99 | 0.89 | 99 | 0.94 |
| Oligomers + AZD103 | 95 | 0.50 | 100 | 0.99 |
| | | | | |
| **In vivo administration** | | | | |
| Baseline | 100 | | 100 | |
| 7 Oligomers alone | 130 | 0.003 | 169 | 0.009 |
| 4 Oligomers + 30 mg/kg | 109 | 0.2 | 120 | 0.15 |
| 5 Oligomers + 100 mg/kg | 100 | 0.9 | 120 | 0.3 |
| 6 Oligomers + 300 mg/kg | 109 | 0.26 | 99 | 0.97 |

The following are citations for publications referred to herein.
1. Selkoe, D.J. Deciphering the genesis and fate of amyloid beta-protein yields novel therapies for Alzheimer disease. J. Clin Invest. 110, 1375-1381 (2002).
2. Wong, P.C., Cai, I.T., Borchelt, D.R & Price, D.L. Genetically engineered mouse models of neurodegenerative diseases. Nat. Neurosci. 5, 633-639 (2002).
3. Nicoll, J.A.R, Wilkinson, D., Holmes, C., Steart, P., Markham, H. & Weller, R.O. Neuropathology of human Alzheimer disease after immunization with amyloid-β peptide: a case report. Nat. Med. 9,448-452 (2003).
4. Hock C. et al., Antibodies against beta-amyloid slow cognitive decline in Alzheimer's disease. Neuron 38, 547-554 (2003).
5. Orgogozo, J.M., et al., Subacute meningoencephalitis in a subset of patients with AD after Aβ42 immunization. Neurology61, 46-54 (2003).
6. Schenk D, et al., Immunization with amyloid-beta attenuates Alzheimer-disease-like pathology in the PDAPP mouse. Nature400, 173-177 (1999).
7. McLaurin, J. et al.,Therapeutically effective antibodies against amyloid-beta peptide target amyloid-beta residues 4-10 and inhibit cytotoxicity and fibrillogenesis. Nat. Med.8, 1263-1269 (2002).
8. Golde, T.E. Alzheimer disease therapy: Can the amyloid cascade be halted? J. Clin. Invest.111, 11-18 (2003).
9. McLaurin, J. & Chakrabartty, A. Membrane disruption by Alzheimer beta-amyloid peptides mediated through specific binding to either phospholipids or gangliosides. Implications for neurotoxicity. J. Biol. Chem.271, 26482-26489 (1996).
10. McLaurin, J. & Chakrabartty, A. Characterization of the interactions of Alzheimer beta-amyloid peptides with phospholipid membranes. Eur. J. Biochem.245, 355-363 (1997).
11. Koppaka, V. & Axelson, P.H. Accelerated accumulation of amyloid beta proteins on oxidatively damaged lipid membranes. Biochemistry 39, 10011-10016 (2000).
12. Mizuno, T. et al., Cholesterol-dependent generation of a seeding amyloid beta-protein in cell culture. J. Biol. Chem. 274, 15110-15114 (1999).
13. Choo-Smith, L.P. & Surewicz, W.K. The interaction between Alzheimer amyloid beta(1-40) peptide and ganglioside GMl-containing membranes. FEBS Lett 402, 95-98 (1997).
14. Yanagisawa, K. et al., GM1 ganglioside-bound amyloid beta-protein (A beta): a possible form of preamyloid in Alzheimer's disease. Nat. Med. 1, 1062-1066 (1995).
15. McLaurin, J. Franklin, T. Chakrabartty, A. & Fraser, P. E. Phosphatidylinositol and inositol involvement in Alzheimer amyloid-beta fibril growth and arrest. J. Mol. Biol. 278, 183-194 (1998).
16. McLaurin, J., Goloumb, R., Jurewicz, A., Antel, J. P. & Fraser, P. E. Inositol stereoisomers stabilize an oligomeric aggregate of Alzheimer amyloid beta peptide and inhibit abeta -induced toxicity. J. Biol. Chem.275, 18495-18502 (2000).
17. Chishti, M. A. et al.,Early-onset amyloid deposition and cognitive deficits in transgenic mice expressing a double mutant form of amyloid precursor protein 695. J.Biol Chem 276, 21562-21570 (2001).
18. Janus,C. et al., Aβ peptide immunization reduces behavioural impairment and plaques in a model of Alzheimer's disease. Nature 408, 979-982 (2000).
19. Morris, R. Development of a water-maze procedure for studying spatial learning in the rat. J. Neurosci Methods 11, 47-60 (1984).
20. Spector, R. Myo-inositol transport through the blood-brain barrier. Neurochemical Research 13, 785-787 (1988).
21. Uldry, M. et al., Identification of a mammalian H(+)-myo-inositol symporter expressed predominantly in the brain. EMBO 20, 4467-4477 (2001).
22. Uldry, M. & Thorens B. The SLC2 family of facilitated hexose and polyol transporters. Pflugers Acta, 447,480-489 (2004).
23. Shetty,H.U. & Hollway, H.W. Assay of myo-inositol in cerebrospinal fluid and plasma by chemical ionization mass spectrometry of the hexaacetate derivative. Biol. Mass Spec. 23, 440-444 (1994).
24. Kayed, R. et al., Common Structure of Soluble amyloid oligomers implies common mechanism of pathogenesis. Science 300, 486-489 (2003).
25. Klein, W.L. Aβ toxicity in Alzheimer's disease: globular oligomers (ADDLs) as a new vaccine and drug targets. Neurochem. Int. 41, 345-352 (2002).
26. Walsh, D.M. et al., Naturally secreted oligomers of amyloid beta protein potently inhibit hippocampal long-term potentiation in vivo. Nature 416, 535-539 (2002a)
27. Walsh, D.M., Klyubin, I., Fadeeva, J.V., Rowan, M.J. & Selkoe, D.J. Amyloid-beta oligomers: their production, toxicity and therapeutic inhibition. Biochem Soc. Trans. 30, 552-557 (2002b).
28. Lambert, M.P. et al., Diffusible, non-fibrillar ligands derived from Abetal-42 are potent central nervous system neurotoxins. Proc. Natl. Acad. Scie USA 95, 6448-6453 (1998).
29. McLean, C.A. et al., Soluble pool of Abeta amyloid as a determinant of severity of neurodegeneration in Alzheimer's disease. Ann Neurol. 46, 860-866 (1999).
30. Kirkitadze, M.D. et al., Paradigms shifts in Alzheimer's disease and other neurodegenerative disorders : the emerging role of oligomeric assemblies. J. Neurosci. Res. 69, 567-577 (2002).
31. Lombardo, J.A., et al., Amyloid-beta antibody treatment leads to rapid normalization ofplaque-induced neuritic alterations. J Neurosci. 23, 10879-10883 (2003).
32. Hsaio, K.K. et al., Age-related CNS disorder and early death in transgenic FVB/N mice overexpressing Alzheimer amyloid precursor proteins. Neuron 15, 1203-1218 (1995).
33. Moechars D., et al., Early Phenotypic Changes in Transgenic Mice That Overexpress Different Mutants of Amyloid Precursor Protein in Brain J. Biol. Chem., 274, 6483 - 6492 (1999).
34. Leissring, M.A. et al., Enhanced proteolysis of β-amyloid in APP transgenic mice prevents plaque formation, secondary pathology and premature death. Neuron 40, 1087-1093 (2003).
35. Phinney, A.L, et al., In vivo reduction of amyloid-beta by a mutant copper transporter. Proc Natl Acad Sci U S A. 100, 14193-14198 (2003).
36. Levine, J. Controlled trials of inositol in psychiatry. Eur. Neuropsychopharmacology 7, 147-155 (1997).
37. Kofman, O. et al., The effect of peripheral inositol injection on rat motor activity models of depression Isr. J. Med Sci. 29, 580-586 (1993).
38. Agam, R. et al., High-dose peripheral inositol raises brain inositol levels and reverses behavioral effects of inositol depletion by lithium Pharmaol. Biochem. Behav. 49, 341-343 (1994).
39. Richards, M. H. & Belmaker, R. H. Epi-inositol is biochemically active in reversing lithium effects on cytidine monophosphorylphosphatidate (CMP-PA) J. Neural Transm 103, 1281-1285 (1996).
40. Tanaka, M. et al., Trehalose alleviates polyglutamine-mediated pathology in a mouse model of Huntington disease. Nat. Med 10, 148-154 (2004).
41. Vaucher, E. et al., Object Recognition Memory and Cholinergic Parameters in Mice Expressing Human Presenilin 1 Transgenes. Exp. Neurol. 175, 398-406 (2002).
42. Mount, H.T.J., Progressive sensorimotor impairment is not associated with reduced dopamine and high energy phosphate donors in a model of ataxia-telangiectasia. J Neurochem 88:1449-1454.
43. Wiltfang, J. et al., Highly conserved and disease-specific patterns of carboxyterminally truncated Abeta peptides 1-37/38/39 in addition to 1-40/42 in Alzheimer's disease and in patients with chronic neuroinflammation J Neurochem 81, 481-496 (2002)
44. Haccou,P.,& Mellis, E., Statistical Analysis of Behavioural Data, pg 120-186, Oxford University Press, Oxford (1995).
45. Sarvey JM, Burgard EC & Decker G. Long-term potentiation: studies in the hippocampal slice. J Neurosci Methods 28,109-124 (1989).
46. Stanton PK & Sarvey JM. Norepinephrine regulates long-term potentiation ofboth the population spike and dendritic EPSP in hippocampal dentate gyrus. Brain Res Bull. 18, 115-119 (1987).
47. Moyer JR Jr, & Brown TH. Methods for whole-cell recording from visually preselected neurons of perirhinal cortex in brain slices from young and aging rats. J Neurosci Methods.86, 35-54 (1998).
48. Phinney, A. et al., No hippocampal neuron or synaptic bouton loss in learning-impaired aged p-amyloid precursor protein-null mice. Neuroscience 90, 1207-1216 (1999).
49. Hu, L. et al., The impact of Aβ-plaques on Cortical Cholinergic and Non-cholinergic Presynaptic boutons in Alzheimer's Disease-like Transgenic mice. Neuroscience 121, 421-432 (2003).
50. Cleary, J.P., Walsh, D.M., Hofmeister, J.J., Shankar, G.M., Kuskowski, M.A., Selkoe, D.J., & Ashe, K.H. (2005) Natural oligomers of the amyloid-β protein specifically disrupt cognitive function. Nature Neuroscience, 8: 79-84.
51. Hsiao, K. K.; Chapman, P.; Nilsen, S.; Eckman, C. B.; Harigaya, Y.; Younkin, S. G.; Yang, F.; Cole, G. M. Correlative memory deficits, Aβ elevation, and amyloid plaques in transgenic mice. Science 274: 99-102; 1996.
52. O'Hare, E., Levine, A.S., Semotuk, M.T., Tierney, K.J., Shephard, R., Grace, M. & Cleary, J. (1996) Utilization of an operant model of food reinforced behavior involving neuropeptide Y, insulin, 2-deoxy-d-glucose, and naloxone. Behavioral Pharmacology, 7: 742-753.
53. Richardson, R.L., Kim, E-M., Shephard, R.A., Gardiner, T., Cleary, J., & O'Hare, E. (2002) Behavioral and histopathological analyses of ibuprofen treatment on the effect of aggregated Aβ1-42 injections in the rat. Brain Research, 54: 1-10.
54. Walsh, D.M., Klyubin, I., Fadeeva, J., Cullen, W., Anwyl, R., Wolfe, M., Rowan, M., Selkoe, D.J. (2002) Naturally secreted oligomers of amyloid-β protein potently inhibit hippocampal long-term potentiation in vivo Nature 516: 535-539.

## Claims

1. *Scyllo*-inositol for use in treating a neuronal disorder such as drug withdrawal or alcoholism.

2. *Scyllo*-inositol according to claim 1, for use in the treatment of drug withdrawal.

3. *Scyllo*-inositol according to claim 1, for use in the treatment of alcoholism.

4. *Scyllo*-inositol for use in restoring long term potentiation in a subject treated for drug withdrawal or alcoholism.

5. The use of *Scyllo*-inositol in the manufacture of a medicament for the treatment of a neuronal disorder such as drug withdrawal or alcoholism in a subject.

6. The use of *Scyllo*-inositol in the manufacture of a medicament for restoring long term potentiation in a subject treated for drug withdrawal or alcoholism.
